# EUROPEAN PATENT APPLICATION

(11) **EP 4 847 498 A1**
(43) Date of publication of application: **02.12.2026**
(21) Application number: 24920552.7
(22) Date of filing: 18.11.2024
(51) Int. Cl.: C07D 401/14, A61K 31/496, A61P 43/00, A61K 47/54, A61K 31/404, A61K 31/4439, A61K 31/501

(54) **PRODRUG COMPOUND ACTIVATED BY FIBROBLAST ACTIVATION PROTEIN**

(30) Priority: 24.01.2024 KR 20240011227; 14.10.2024 KR 20240139751
(71) Applicant: Seoul National University R&DB Foundation, Seoul 08826 (KR); Seoul National University Hospital, Seoul 03080 (KR)
(72) Inventor: YU, Jiyeon, Suwon-si Gyeonggi-do 16509 (KR); IM, Hyung-Jun, Suwon-si Gyeonggi-do 16229 (KR); LEE, Byung Chul, Hanam-si Gyeonggi-do 13014 (KR)
(74) Representative: Müller, Christian Stefan Gerd
(86) International application number: PCT/KR2024/018142
(87) International publication number: WO 2025/159301

(57) **Abstract**

The present invention relates to a prodrug compound activated by a fibroblast activation protein (FAP).

## Description

### Technical Field

The present invention relates to a prodrug compound that is activated by fibroblast activation protein (FAP) and the use thereof as an antifibrotic agent.

### Background Art

It has been reported that fibroblast activation protein (FAP) is generally rarely expressed in normal adult tissues, but is selectively and highly expressed in more than 90% of reactive stromal fibroblasts of examined epithelial malignancies (primary and metastatic), including epithelial malignancies (primary and metastatic), including lung, colorectal, bladder, ovarian and breast carcinomas, and in malignant mesenchymal cells of bone and soft tissue sarcomas. It is known that fibroblast activation protein (FAP) is involved in tissue remodeling and is highly expressed in activated fibroblasts in scar tissue, in addition to tumor or cancer tissue. Recently, it has been revealed that fibroblast activation protein (FAP) is highly expressed in fibrotic lesions in tissues such as the liver, lungs, and colon.

Meanwhile, FAP-targeted prodrugs have been developed for the purpose of diagnosing or treating diseases associated with fibroblast activation protein (FAP). However, these prodrugs have limitations in that they are not specific only to FAP, due to the high homology of FAP and DPP4 around the active site and the considerable overlap in FAP and PREP substrate specificity. For this reason, there is a problem in that the drug can be activated in various unwanted human tissues where DPP4 and PREP are expressed. Additionally, until now, the focus has been only on prodrugs that use FAP as a biomarker for tumor tissue, and no FAP-targeted antifibrotic agents have been developed.

### DISCLOSURE

### Technical Problem

An object of the present invention is to provide a prodrug compound with a novel structure, which, by having high selectivity for fibroblast activation protein (FAP), may be specifically released and delivered only in a fibrotic environment where FAP overexpression occurs.

Another object of the present invention is to provide a pharmaceutical composition comprising the prodrug, which is able to effectively prevent, alleviate, or treat a fibrosis-related disease.

However, the technical problems to be solved by the present invention are not limited to the problems mentioned above, and other problems not mentioned may be clearly understood by those skilled in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

The present invention provides a prodrug that may be activated by fibroblast activation protein (FAP). The prodrug of the present invention is characterized by comprising: a substrate that may be recognized and cleaved by FAP; and an oxindole-based kinase inhibitor drug, wherein the substrate and the drug are linked to each other by a linker having a p-aminobenzyl structure.

In the present specification, "fibroblast activation protein (FAP)" is an enzyme belonging to the serine protease family that plays an important role in tissue remodeling, wound healing, and certain pathological conditions such as cancer. FAP is rarely expressed in normal adult tissues but is expressed at high levels in cancer, fibrosis, inflammatory diseases, etc.

In the present specification, "prodrug" means a compound that may be converted under physiological conditions or by solvolysis to a biologically active compound described herein. Thus, the term "prodrug" refers to a precursor of a biologically active compound that is pharmaceutically acceptable. In some embodiments, a prodrug may be inactive when administered to a subject, but is converted to an active compound, for example, by hydrolysis. The prodrug compound often offers advantages of solubility, tissue compatibility or delayed release in a mammalian organism

In general, in prodrug design, the selection of a substrate, a drug, and a linker connecting them is very important. This is because each component constituting the prodrug may have a significant impact on the drug's efficacy, stability, and overall therapeutic effect. In particular, because a drug used as a payload in the present invention contains an oxindole ring, the polar and electronic properties of this ring may promote the degradation of the linker.

The inventors of the present invention have made extensive efforts, and as a result, developed a prodrug compound in which a drug containing an oxindole ring is attached to an FAP substrate by a linker represented by the formula below, that is, a p-aminobenzyl linker. When the substrate of the prodrug of the present invention is cleaved by FAP, the linker of the structure is subsequently degraded and at the same time, an oxindole-based kinase inhibitor drug is released and may exert pharmacological activity. On the other hand, in normal tissues where the substrate is not cleaved by FAP, the linker is not degraded, and thus the structure of the prodrug is maintained stably and the drug may remain in an inactive state. Hereinafter, the present invention will be described in detail.

According to one embodiment of the present invention, the present invention relates to a prodrug compound selected from the group consisting of a compound represented by Formula 1 below, a pharmaceutically acceptable salt thereof, a solvate thereof, and a hydrate thereof:

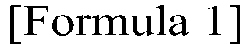

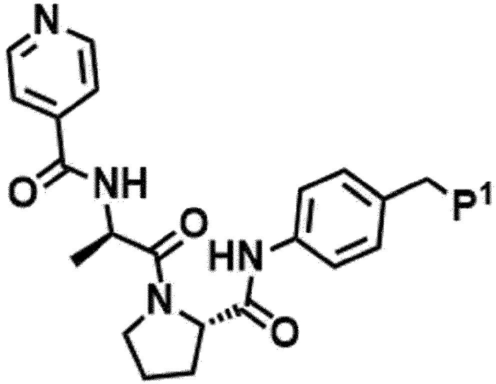

In Formula 1 above, P¹ is a radical of a kinase inhibitor drug containing an oxindole ring, which is a radical derived by removal of a hydrogen atom from the NH group of the oxindole ring. Specifically, the radical structure P¹ of the oxindole-based kinase inhibitor may be selected from the formulas below, without being limited thereto. In the formulas below, * denotes a portion through which linking takes places.

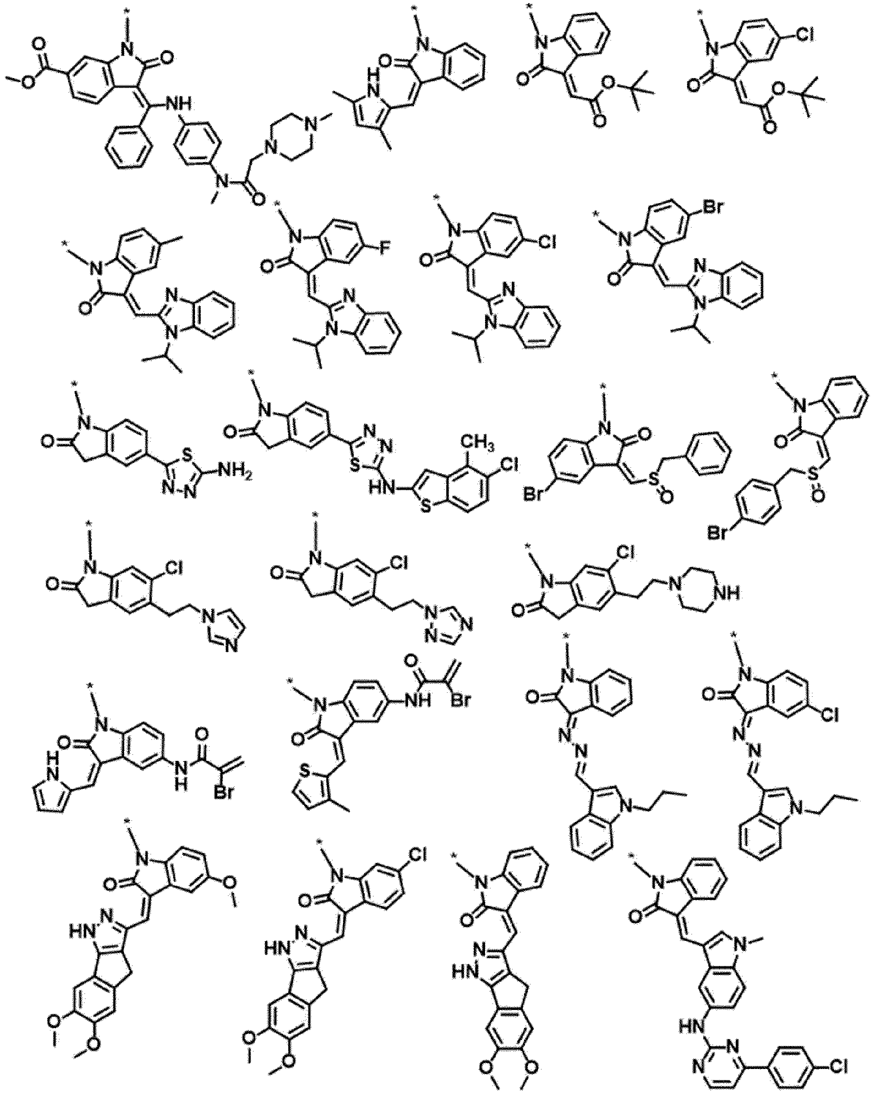

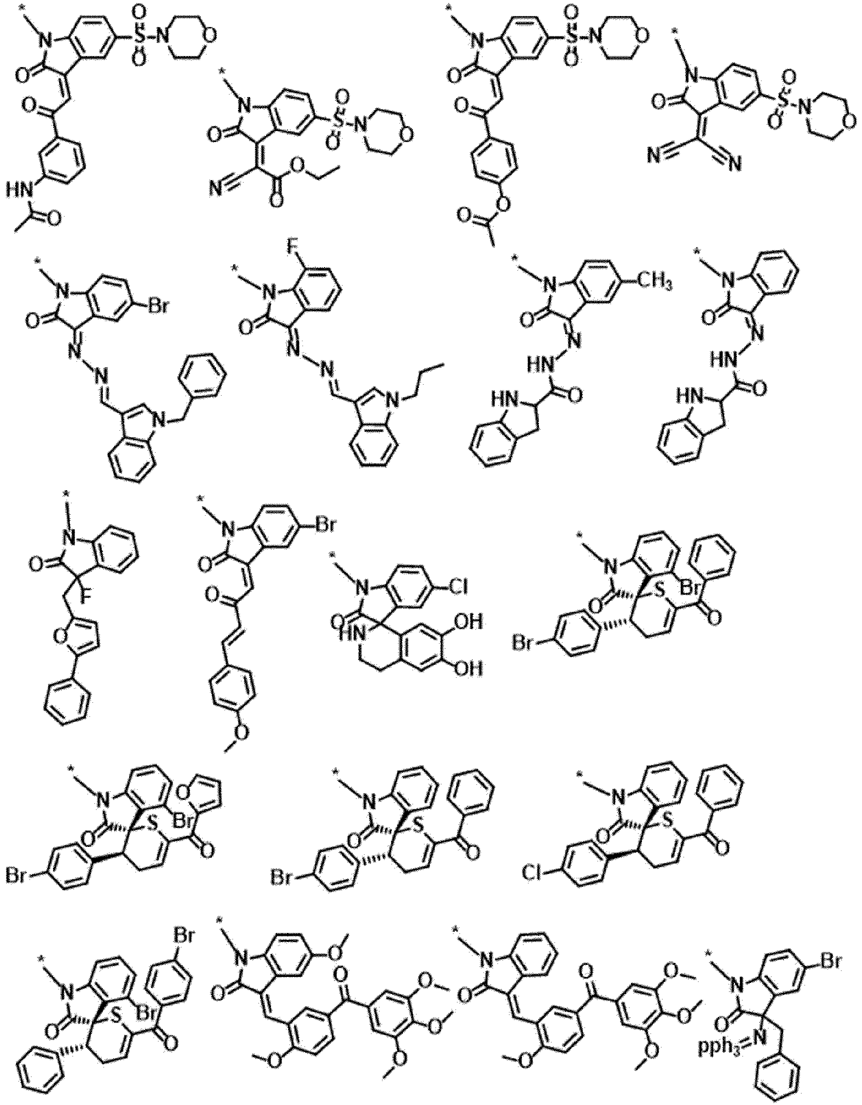

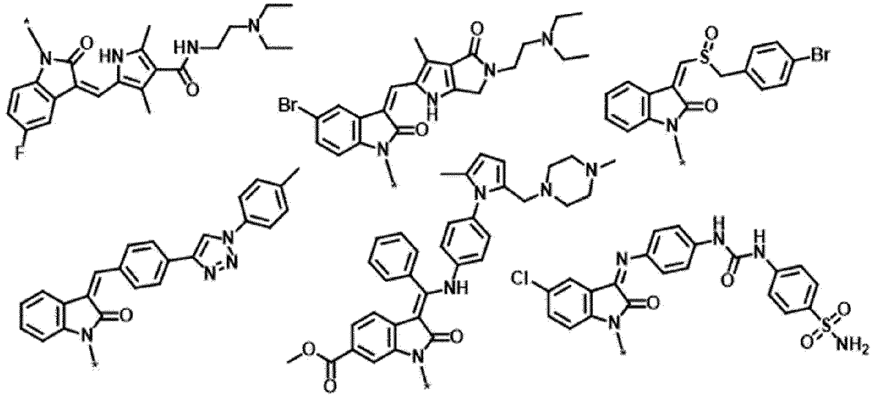

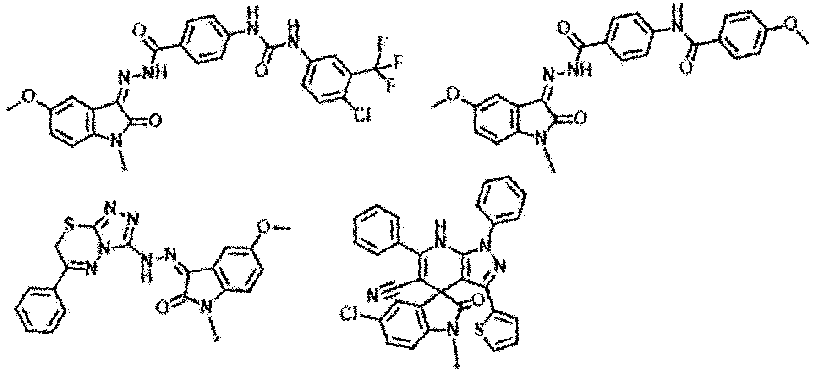

The oxindole ring-containing compound represented by the above formula acts as a kinase inhibitor (preferably a tyrosine kinase inhibitor) and is capable of treating fibrosis (Biomed Pharmacother. 2021 Sep;141:111842.; J. Med. Chem. 58 (2015) 1053e1063; Molecules 2017, 22(11), 1979). In particular, the effects of nintedanib on the alleviation or treatment of fibrosis are found in various papers and clinical studies (J Med Chem. 2015 Feb 12;58(3):1053-63); Expert Opin Pharmacother. 2018 Feb;19(2):167-175.; Eur Respir J. 2015 May;45(5):1434-45).

However, the prodrug compound represented by Formula 1 provided by the present invention has a novel structure that is not known.

The compound represented by Formula 1 may be selected from the group consisting of the following compounds:

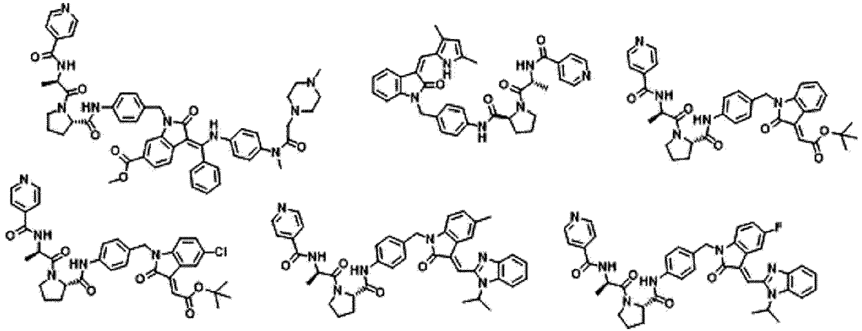

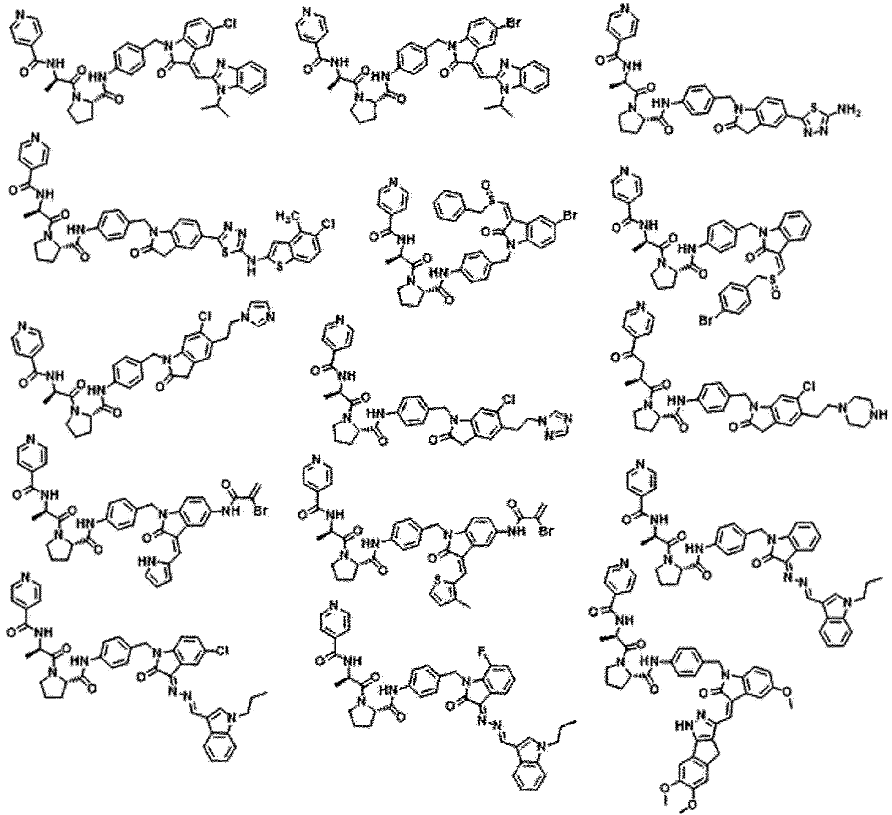

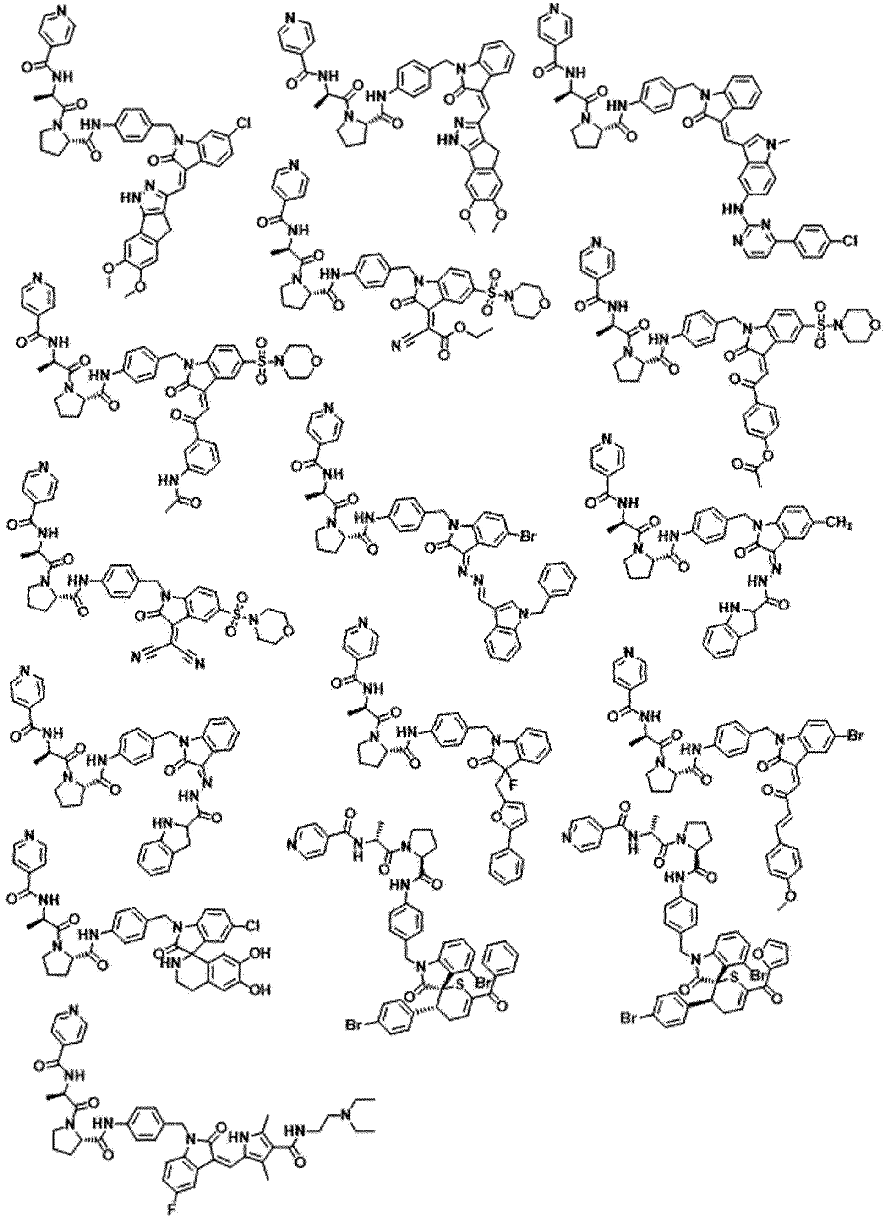

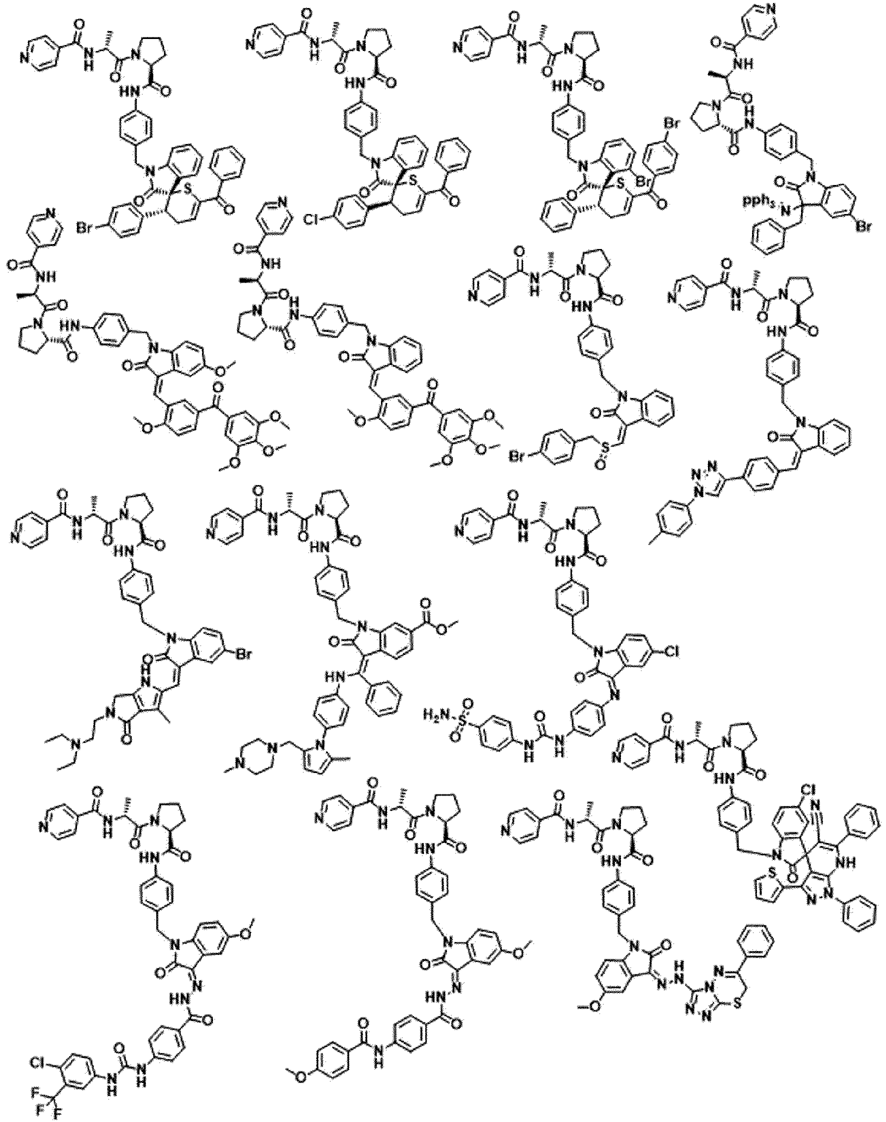

Preferably, the prodrug compound may be a compound represented by Formula 8 below, without being limited thereto:

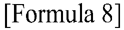

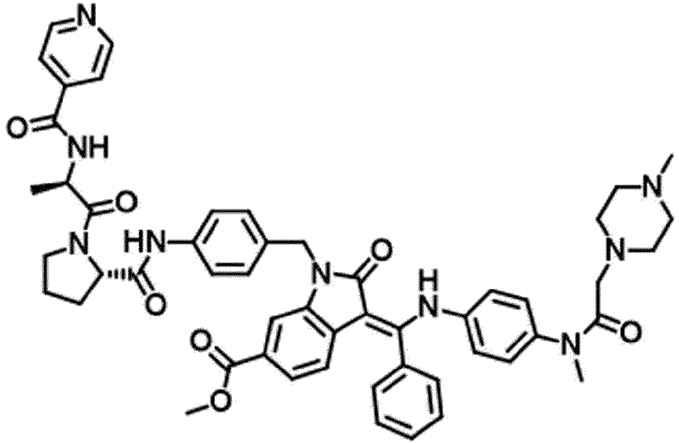

In prodrugs, the linker must not only serve to provide a functional and physical linkage between the ligand and the drug, but also be degraded by a reaction occurring immediately after cleavage of the substrate so that the drug can be active. Although various types of self-immolative linkers have been known, there have been cases where the drug fails to exhibit effective activity because, structurally, the FAP substrate is not properly recognized by FAP or the linker is not properly removed after cleavage of the FAP substrate. In addition, there has been a problem in that the exposed end of the linker reacts with other parts of the drug or the cleaved FAP substrate, preventing the drug from acting effectively.

In the prodrug compound of the present invention, the drug that binds to the ligand, particularly the oxindole-based kinase inhibitor drug, has a large structure. Thus, when the drug binds directly to the ligand that is recognized and cleaved by FAP without a linker, FAP cannot properly recognize the ligand due to steric hindrance and thus cannot exert enzyme activity.

However, according to the present invention, an N-(1-(2-carbamoylpyrrolidin-1-yl)-1-oxopropan-2-yl)isonicotinamide moiety as an FAP substrate and an oxindole-based kinase inhibitor drug such as nintedanib are linked to each other by a p-aminobenzoyl linker, so that the linker may easily enter the pocket in the FAP enzyme. Thereby, the substrate may be selectively cleaved by FAP with high sensitivity, and the linker may be subsequently degraded and removed from the drug, allowing the drug P¹ to be active.

More specifically, in the compound according to the present invention, an oxindole-based kinase inhibitor drug such as nintedanib, which is effective against fibrosis, is in an inactive state in normal tissues in which fibroblast activation protein (FAP) is not expressed. However, in an environment where fibroblast activation protein (FAP) is overexpressed, i.e., in fibrotic tissue, the N-(1-(2-carbamoylpyrrolidin-1-yl)-1-oxopropan-2-yl)isonicotinamide moiety is cleaved by FAP as shown in Reaction Schemes 1 and 2 below. Subsequently, the p-aminobenzoyl linker is removed spontaneously, so that the drug may be effectively delivered to the fibrotic tissue and may exert its effective efficacy.

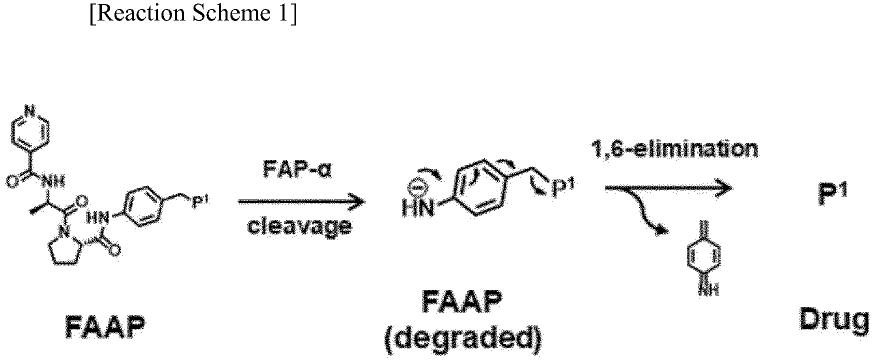

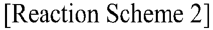

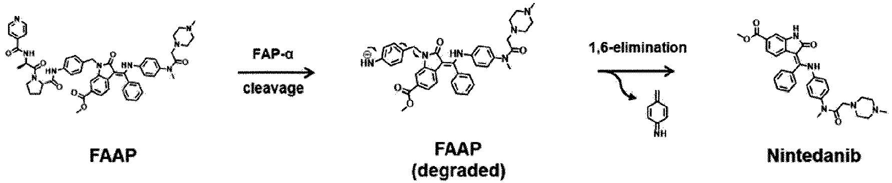

When the prodrug of the present invention reacts with tyrosine kinase such as VEGFR2, FGFR1, PDGFRα, FLT1, etc., the prodrug is located outside the binding pocket. For this reason, the binding affinity between the drug P¹ and the tyrosine kinase is lowered, so the drug may not exhibit effective activity. Therefore, the prodrug of the present invention has a low likelihood of side effects because the drug does not act properly in normal tissues.

In the present invention, the fibrotic tissue may be a liver, lung, heart, vascular system, articular or interstitial tissue, pancreas, skin, mouth, digestive tract, brain, breast, bone marrow, peritoneum, or kidney tissue in which a fibrotic condition has occurred, and preferably may be pulmonary fibrotic tissue, without being limited thereto.

The present invention also provides pharmaceutically acceptable salts of the above-described compounds. The pharmaceutically acceptable salts are salts which are usually considered by those skilled in the art to be suitable for medical applications, e.g., because they are not harmful to subjects which may be treated with the salts, or which give rise to side effects which are tolerable within the respective treatment. Usually, the pharmaceutically acceptable salts are salts which are considered as acceptable by the regulatory authorities, such as the US Food and Drug Administration (FDA), the European Medicines Agency (EMA), or the Japanese Ministry of Health, Labor and Welfare Pharmaceuticals and Medical Devices Agency (PMDA). However, the present invention in principle also encompasses salts of the compounds according to the present invention which are as such not pharmaceutically acceptable, e.g., as intermediates in the production of the compounds according to the present invention or physiologically functional derivatives thereof, or as intermediates in the production of pharmaceutically acceptable salts of the compounds according to the present invention or physiologically functional derivatives thereof. Said salts include water-insoluble salts and, particularly, water-soluble salts.

In each case, those skilled in the art can readily determine whether a certain compound according to the present invention or a physiologically functional derivative thereof can form a salt, i.e., whether the compound according to the present invention or a physiologically functional derivative thereof has a group which may carry a charge, such as, for example, an amino group, a carboxylic acid group, etc.

Exemplary salts of the compounds of the present invention are acid addition salts or salts with bases, particularly pharmaceutically acceptable inorganic and organic acid addition salts and salts with bases customarily used in pharmacy, which are either water insoluble or, particularly, water-soluble acid addition salts. Salts with bases may, depending on the substituents of the compounds of the present invention, also be suitable. Acid addition salts may, for example, be formed by mixing a solution of a compound of the present invention with a solution of a pharmaceutically acceptable acid such as hydrochloric acid, sulfuric acid, fumaric acid, maleic acid, succinic acid, acetic acid, benzoic acid, citric acid, tartaric acid, carbonic acid, or phosphoric acid. Likewise, pharmaceutically acceptable base addition salts may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, arginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, digluconate, dihydrochloride, dodecylsulfate, edetate, edisylate, ethanesulfonate, formate, fumarate, galactate, galacturonate, gluconate, glutamate, glycerophosphate, hemisulfate, heptanoate, hexanoate, hexylresorcinate, hydrobromide, hydrochloride, hydroiodide, 2-hydroxy-ethanesulfonate, hydroxynaphthoate, iodide, isobutyrate, isothionate, lactate, laurate, lauryl sulfate, malate, maleate, malonate, mandelate, methanesulfonate (mesylate), methylsulfate, 2-naphthalenesulfonate, nicotinate, nitrate, oleate, oxalate, palmitate, pantothenate, pectinate, persulfate, 3-phenylpropionate, phosphate/diphosphate, phthalate, picrate, pivalate, polygalacturonate, propionate, salicylate, stearate, sulfate, suberate, succinate, tannate, tartrate, tosylate, undecanoate, valerate, and the like.

Salts, which are not pharmaceutically acceptable and which can be obtained, for example, as process products during the production of the compounds according to the present invention on an industrial scale, are also encompassed by the present invention and, if desired, may be converted into pharmaceutically acceptable salts by processes known to those skilled in the art.

In addition, the compounds of the present invention as well as salts thereof may contain various amounts of solvent when isolated, for example, in crystalline form. Therefore, solvates and in particular, hydrates of the compounds of the present invention, and also solvates and in particular, hydrates of the salts of the compounds of the present invention may be included within the scope of the present invention. More particularly, the present invention may include hydrates of the compounds, salts, and/or physiologically functional derivatives according to the present invention, which comprise one, two or one half water molecule, with respect to their stoichiometry.

According to another embodiment of the present invention, the present invention relates to a pharmaceutical composition for preventing, alleviating or treating fibrotic disease, comprising, as an active ingredient, a prodrug compound provided by the present invention.

In the present invention, the fibrotic disease may be, but is not limited to, scleroderma, atherosclerosis, cardiac fibrosis, organ transplant fibrosis, muscular fibrosis, pancreatic fibrosis, myelofibrosis, hepatic fibrosis, splenic fibrosis, pulmonary fibrosis, idiopathic pulmonary fibrosis, idiopathic interstitial fibrosis, diffuse interstitial fibrosis, interstitial lung disease, chronic interstitial lung disease, pneumoconiosis, silicosis, interstitial fibrosis, sarcoidosis, mediastinal fibrosis, cardiac fibrosis, atrial fibrosis, endocardial fibrosis, renal fibrosis, macular degeneration, keloid lesions, hypertrophic scars, nephrogenic systemic fibrosis, injection fibrosis, fibrotic complications of surgery, fibrotic chronic allograft vasculopathy, ischemic reperfusion injury-associated fibrosis, arthrofibrosis, Dupuytren's disease, fibrous proliferative lesions of the oral cavity, fibrotic intestinal stenosis, glial scar formation, leptomeningeal fibrosis, fibrosis due to radiation exposure, fibrosis due to mammary cystic rupture, myelofibrosis, retroperitoneal fibrosis, or progressive massive fibrosis, preferably idiopathic pulmonary fibrosis.

In the present invention, the "pharmaceutical composition" may be characterized in that it is in the form of a capsule, tablet, granule, injectable preparation, ointment, powder, or beverage, and the pharmaceutical composition may be characterized in that it is intended for animals, specifically humans.

For use, the pharmaceutical composition may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition according to the present invention may contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preservative, a pain-relieving agent, a solubilizer, an isotonic agent, a stabilizer, and the like may be used in admixture for injections; and a base, an excipient, a lubricant, a preservative, and the like may be used for topical administration. The pharmaceutical composition of the present invention may be prepared in various dosage forms by being mixed with the pharmaceutically acceptable carriers as described above. For example, for oral administration, the pharmaceutical composition may be prepared in the form of tablets, troches, capsules, elixirs, suspensions, syrups, wafers, or the like. For injection, the pharmaceutical composition may be prepared in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, or mineral oil. In addition, the pharmaceutical composition of the present invention may further contain a filler, an anticoagulant, a lubricant, a wetting agent, a fragrance, an emulsifier, a preservative, or the like.

In the present invention, the pharmaceutical composition may further comprise a radical scavenger in addition to the compound of the present invention or a pharmaceutically acceptable salt thereof. The radical scavenger may be used to prevent radiolysis. Radiolysis is the process in which the ionization of oxygen or water molecules induced by the radionuclide leads to the formation of other reactive species, such as superoxide, hydrogen peroxide, hydrogen radicals, ozone and hydroxyl radicals. These reactive species can further cause damage to DNA and other cellular structures. In some embodiments, the radical scavenger is an antioxidant selected from carnosic acid, green tea extract, apigenin, diosmine, rosmarinic acid, lipoic acid, beta carotene, L-ascorbic acid (vitamin C), N-acetyl cysteine (NAC), δ-tocopherol, rutin, amifostine, resveratrol, gentisic acid, and gallic acid. In some embodiments, the radical scavenger may be an antioxidant selected from gallic acid, L-ascorbic acid and N-acetyl cysteine (NAC), without being limited thereto.

The routes of administration of the pharmaceutical composition according to the present invention include, but are not limited to, oral, intravenous, intramuscular, intraarterial, intramedullary, intradural, intracardiac, transdermal, subcutaneous, intraperitoneal, intranasal, enteral, topical, sublingual, or rectal administration. Oral or parenteral administration is preferred. The "parenteral" includes subcutaneous, transdermal, intravenous, intramuscular, intra-articular, intra-synovial, intrasternal, intradural, intra-lesional and intra-cranial injection or infusion techniques. The pharmaceutical composition of the present invention may also be administered in the form of suppositories for intrarectal administration.

In addition, the pharmaceutical composition may vary depending on a variety of factors, including the activity of a specific compound used, the patient's age, body weight, general health status, sex and diet, the time of administration, the route of administration, the rate of excretion, drug combination, and the severity of a specific disease to be prevented or treated. Although the dose of the pharmaceutical composition may vary depending on the patient's condition and body weight, the severity of disease, the drug form, and the route and duration of administration, it may be appropriately selected by those skilled in the art, and the pharmaceutical composition may be administered at a dose of 0.0001 mg/kg/day to 50 mg/kg/day or 0.001 mg/kg/day to 50 mg/kg/day. The administration may be made once a day or several times a day. The dose is not intended to limit the scope of the present invention in any way. The pharmaceutical composition according to the present invention may be formulated in the form of pills, sugar-coated tablets, capsules, liquids, gels, syrups, slurries, or suspensions.

The pharmaceutical composition of the present invention may be administered alone or in combination with other antifibrotic agents. Here, the other antifibrotic agents include, but are not limited to, pirfenidone or receptor tyrosine kinase inhibitors (RTKIs) such as Sorafenib and other RTKIs, or angiotensin II (AT1) receptor blockers, or CTGF inhibitor, or any antifibrotic compound susceptible to interfere with the TGFβ and BMP-activated pathways including activators of the latent TGFβ complex such as MMP2, MMP9, THBS1 or cell-surface integrins, TGFβ receptors type I (TGFBRI) or type II (TGFBRII) and their ligands such as TGFβ, Activin, inhibin, Nodal, anti-Müllerian hormone, GDFs or BMPs, auxiliary co-receptors (also known as type III receptors), or components of the SMAD-dependent canonical pathway including regulatory or inhibitory SMAD proteins, or members of the SMAD-independent or non-canonical pathways including various branches of MAPK signaling, TAK1, Rho-like GTPase signaling pathways, phosphatidylinositol-3 kinase/AKT pathways, TGFβ-induced EMT process, or canonical and non-canonical Hedgehog signaling pathways including Hh ligands or target genes, or any members of the WNT, or Notch pathways which are susceptible to influence TGFβ.

According to another embodiment of the present invention, the present invention relates to a method for preventing, alleviating or treating fibrotic disease, comprising a step of administering a pharmaceutically effective amount of the prodrug compound of the present invention to a subject in need of administration.

In the present invention, the subject is a subject having or suspected of having fibrotic disease. The subject suspected of having the disease refers to all animals that have or may develop the disease, including humans, monkeys, cows, horses, sheep, pigs, chickens, turkeys, quails, cats, dogs, mice, rats, rabbits, or guinea pigs, but includes, without limitation, subjects that can be treated with the active ingredient provided by the present invention.

In the present specification, the term "pharmaceutically effective amount" is an amount sufficient to stop or alleviate the physiological effects caused by fibrosis-related disease in a subject or patient. The appropriate effective amount can be decided by the attending physician within the scope of sound medical judgment, and may be administered once or in several divided doses. However, for the purposes of the present invention, the specific therapeutically effective amount for a particular patient is preferably applied differently depending on various factors, including the type and degree of response to be achieved, the specific composition containing the active ingredient, including whether other agents are used as the case may be, the patient's age, body weight, general health status, sex, and diet, the time of administration, the route of administration, the frequency of administration, the secretion rate of the composition comprising the active ingredient, the duration of treatment, and drugs used simultaneously or in combination with the specific composition, and similar factors well known in the medical field.

The total effective amount of the composition of the present invention may be administered to a patient as a single dose, or may be administered using a fractionated treatment protocol in which multiple doses are administered over a more prolonged period of time. The composition of the present invention may have varying contents of the active ingredient depending on the severity of the disease. Specifically, a preferred total dose of the composition of the present invention may be about 0.0001 mg to 500 mg per kg of patient body weight per day. However, the effective dose of the composition for a patient is determined in view of various factors such as the patient's age, body weight, health status, sex, severity of the disease, diet, and excretion rate, as well as the route of administration of the pharmaceutical composition and the number of treatments with the pharmaceutical composition. Considering these factors, a person skilled in the art will be able to determine an appropriate effective dose according to the specific use of the composition of the present invention. The pharmaceutical composition according to the present invention is not particularly limited in its formulation, administration route, and administration mode, as long as it exhibits the effects of the present invention.

In addition, in the present invention, the method for preventing, alleviating or treating fibrotic disease may be a combination therapy that further comprises administering one or more compounds or substances having therapeutic activity against the diseases.

In the present invention, the "combination" should be understood to refer to simultaneous, individual, or sequential administration. When the administration is done in a sequential or individual manner, the second component should be administered at intervals such that beneficial effects of the combination are not lost.

In the method for preventing, alleviating or treating fibrotic disease, details regarding the type of fibrotic disease or the second component that may be co-administered overlap with those described above, and thus the description thereof will be omitted below.

### Advantageous Effects

According to the prodrug compound provided by the present invention, in a fibrotic environment where fibroblast activation protein (FAP) is overexpressed, particularly in pulmonary fibrotic tissue, the substrate is cleaved by FAP, and the linker is subsequently degraded, so that the oxindole-based drug may be released and exhibit effective activity in lesion tissue. In normal tissues where fibroblast activation protein (FAP) is not expressed or is underexpressed, the prodrug compound of the present invention remains structurally stable, so that the drug is not activated and binding to tyrosine kinase is also prevented, indicating that the prodrug has a low likelihood of side effects.

### Brief Description of Drawings

FIG. 1 shows the results of performing LC-MS analysis on FAAP synthesized in an example of the present invention.
FIG. 2 shows the results of performing NMR analysis (¹H NMR, 400 MHz, CD₃OD) on FAAP synthesized in an example of the present invention.
FIG. 3 shows the results of observing the change in fluorescence intensity after treating an AMC-conjugated FAP substrate according to the present invention with each of FAP, PREP, and DPP4 in Experimental Example 1.
FIG. 4a is a schematic diagram showing the degradation of FAAP over time after treating FAAP according to the present invention with FAP in Experimental Example 2.
FIG. 4b shows the results of analyzing the degree of FAAP degradation over time by HPLC after treating FAAP according to the present invention with FAP in Experimental Example 2.
FIGS. 5a to 5c show the results of analyzing, by LC-MS analysis, whether a precursor would be formed and whether nintedanib would be released due to FAAP degradation over time after FAAP according to the present invention was treated with FAP in Experimental Example 2. Specifically, FIG. 5a shows the results of analyzing the presence of FAAP (①), FIG. 5b shows the results of analyzing the presence of a precursor (②), and FIG. 5c shows the results of analyzing the presence of nintedanib (③).
FIG. 6a shows confocal microscope images of U87MG cells after treatment with nintedanib, FAAP, or an FAP inhibitor (OncoFAP) in Experimental Example 5, and FIGS. 6b and 6c show the results of comparing fluorescence intensity following each treatment.

### Best Mode

According to one embodiment of the present invention, the present invention relates to a prodrug compound selected from the group consisting of a compound represented by Formula 1, a pharmaceutically acceptable salt thereof, a solvate thereof, and a hydrate thereof:

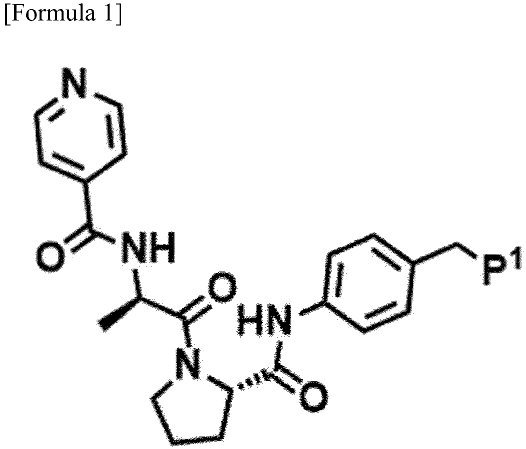

In Formula 1 above, P¹ is a radical of a kinase inhibitor drug containing an oxindole ring, which is a radical derived by removal of a hydrogen atom from the NH group of the oxindole ring. Specifically, the radical structure P¹ of the oxindole-based kinase inhibitor may be selected from the formulas below, without being limited thereto. In the formulas below, * denotes a portion through which linking takes places.

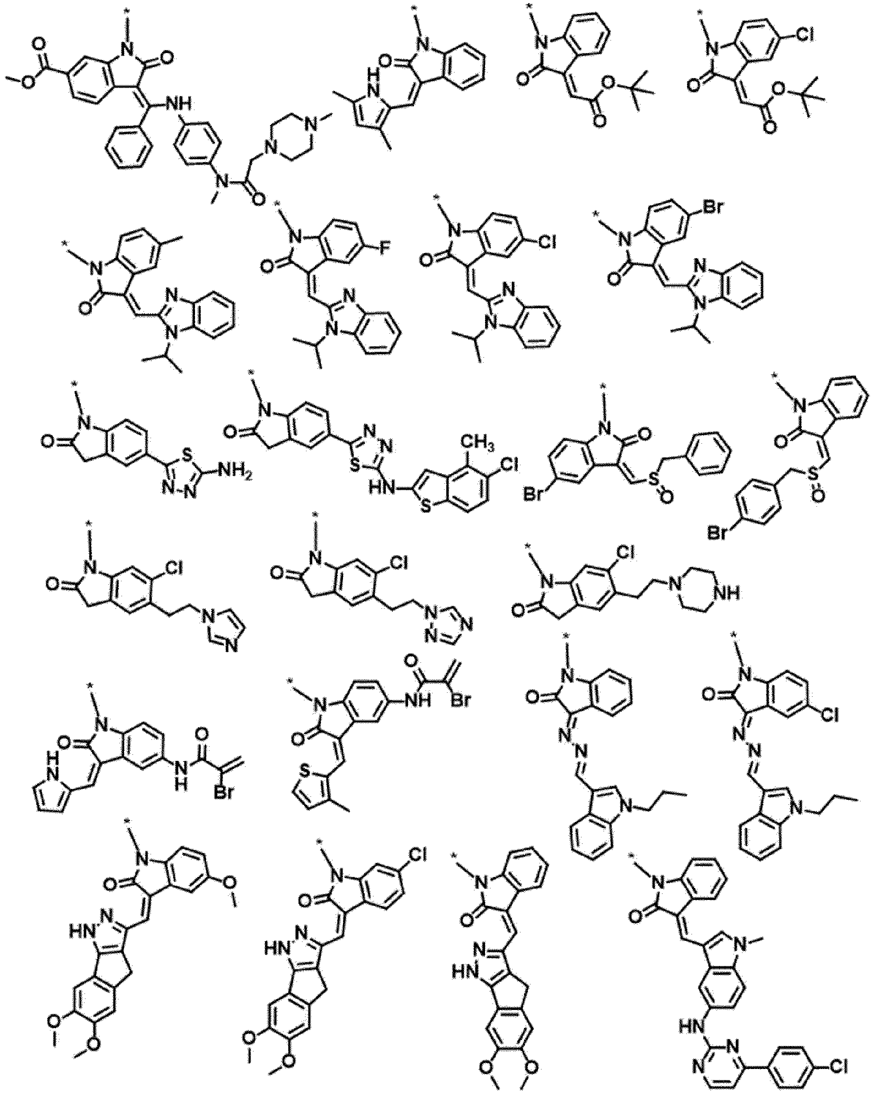

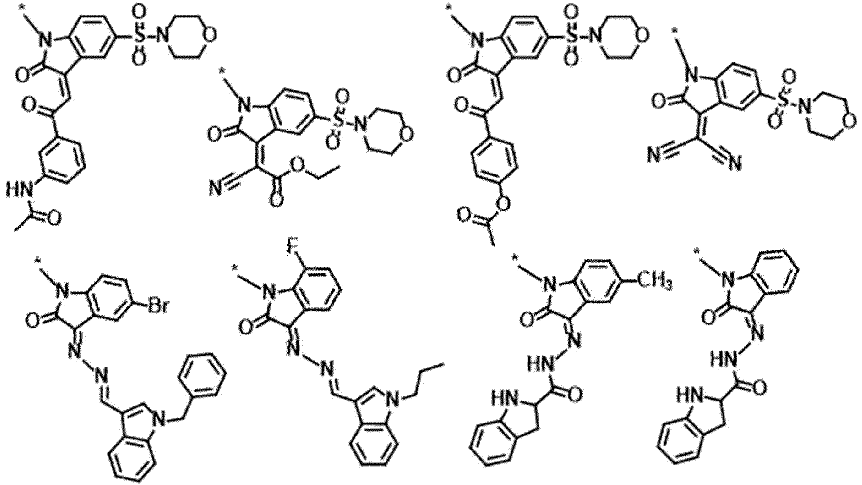

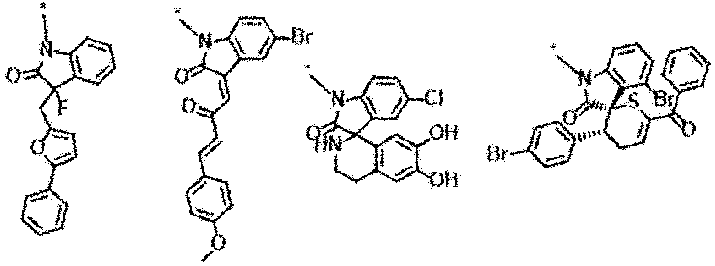

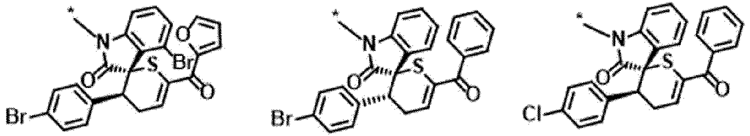

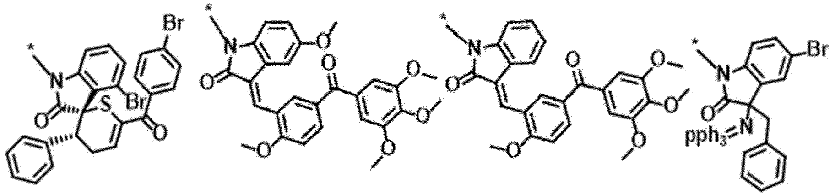

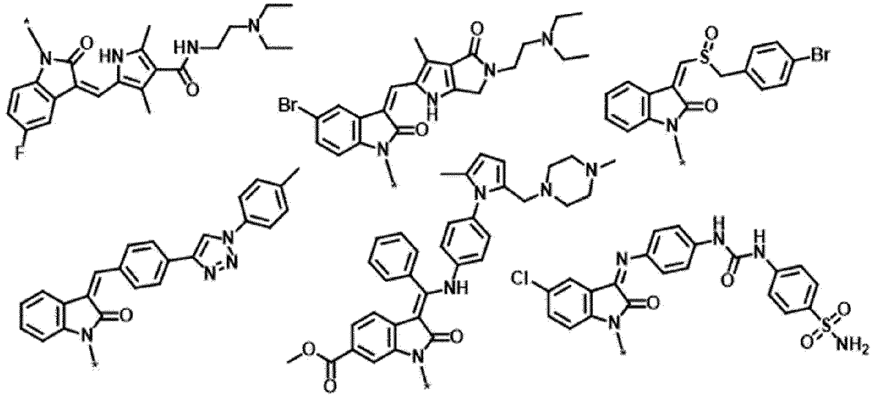

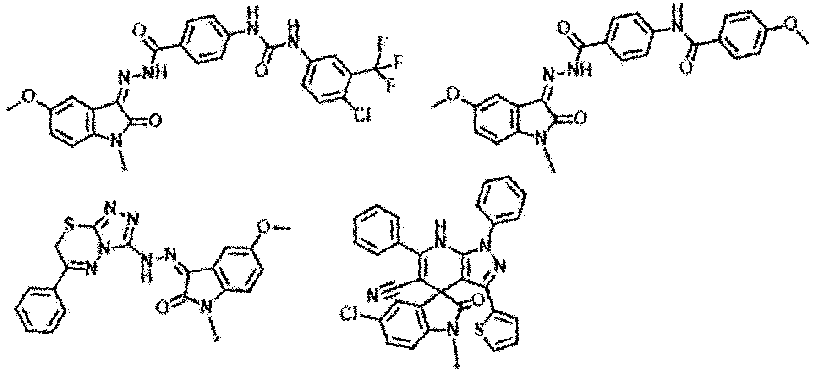

The compound represented by Formula 1 may be selected from the group consisting of the following compounds:

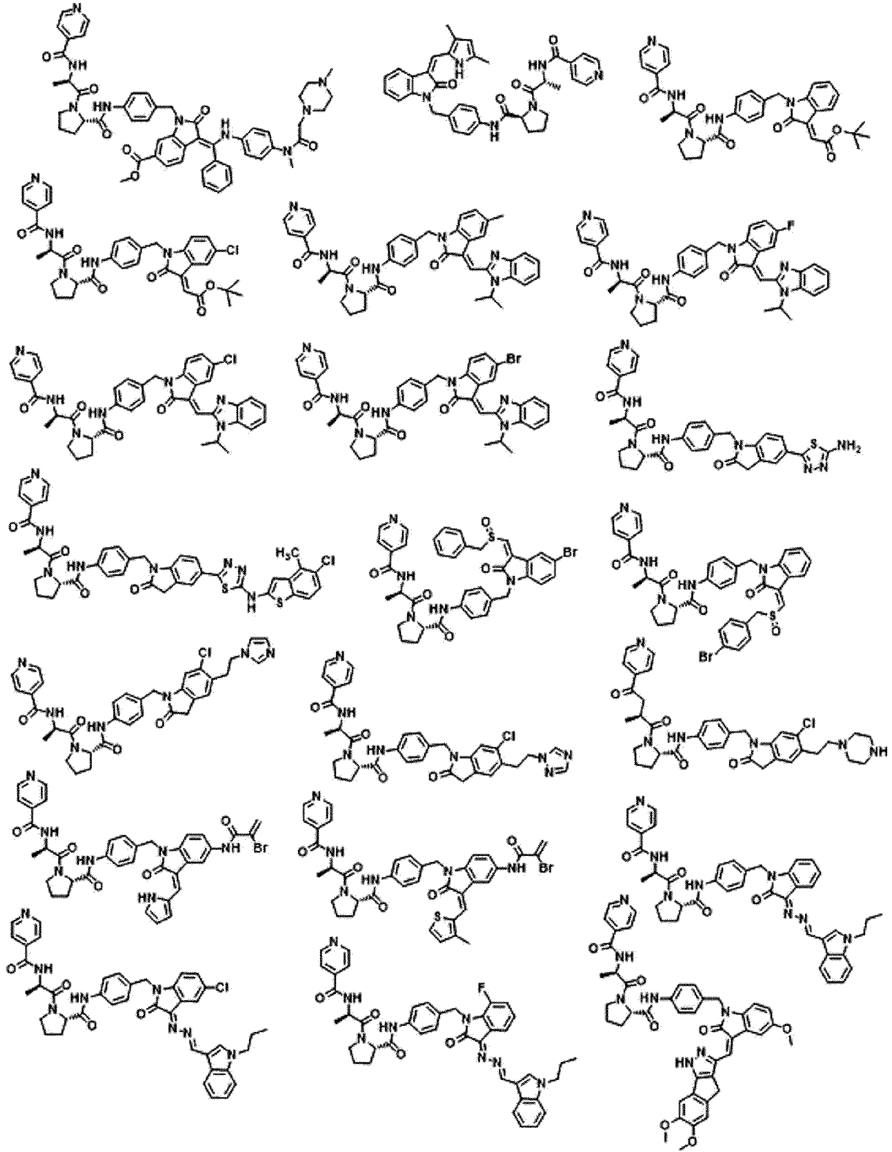

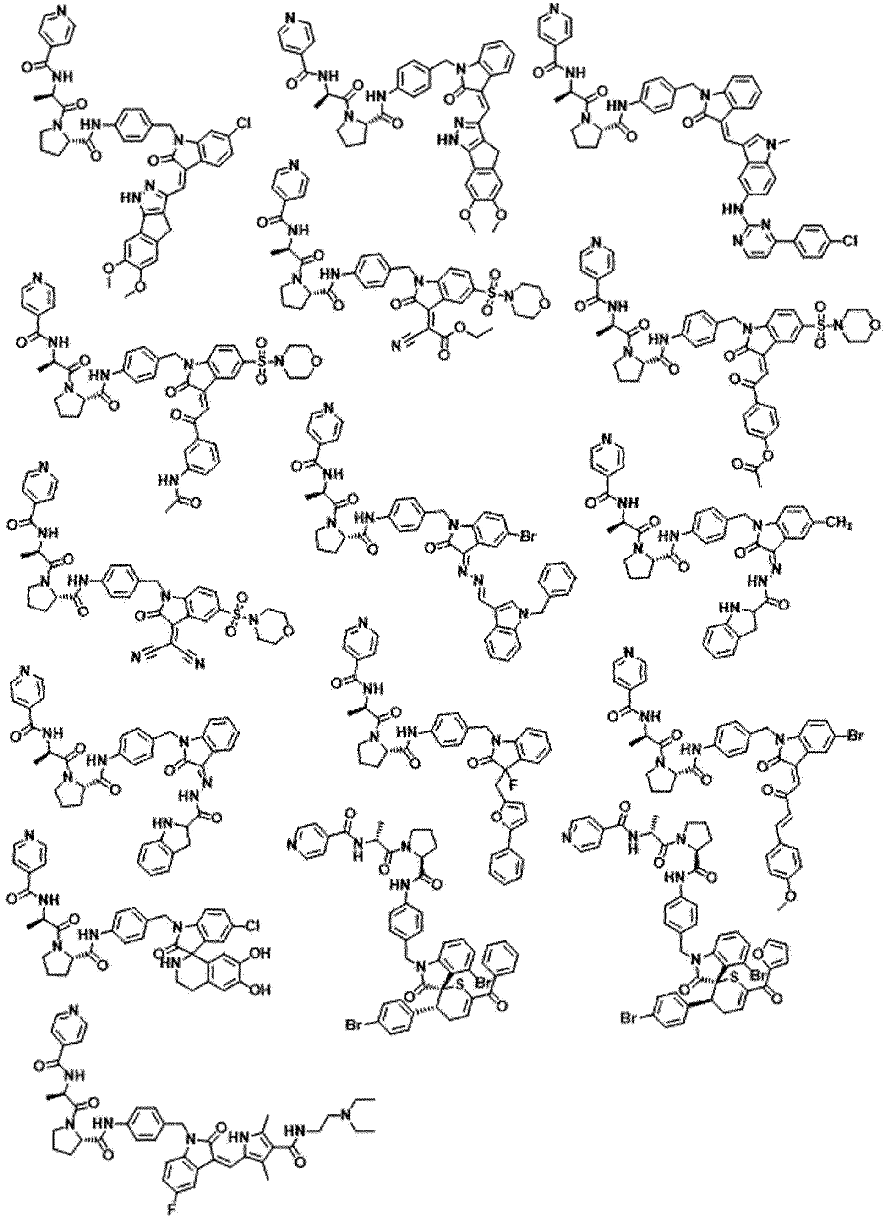

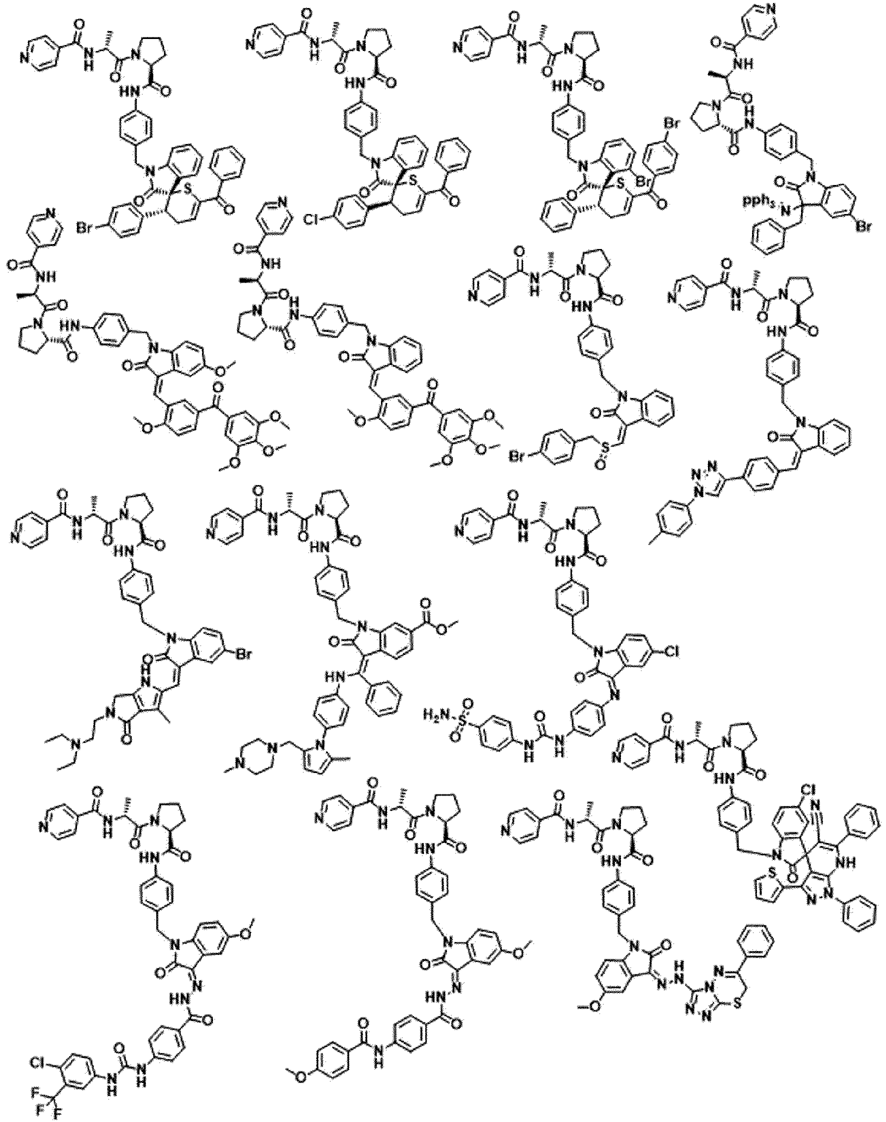

Preferably, the prodrug compound may be a compound represented by Formula 8 below, without being limited thereto:

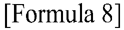

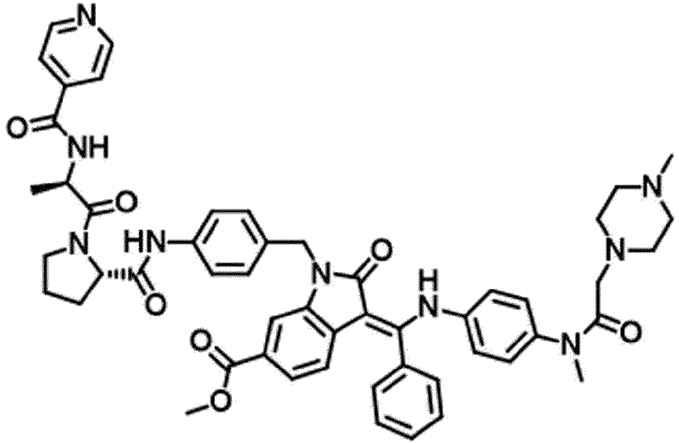

In an environment where fibroblast activation protein (FAP) is overexpressed, i.e., in fibrotic tissue, the N-(1-(2-carbamoylpyrrolidin-1-yl)-1-oxopropan-2-yl)isonicotinamide moiety in the prodrug according to the present invention is cleaved by FAP, and subsequently the p-aminobenzoyl linker is removed spontaneously, so that the drug may be effectively delivered to the fibrotic tissue and exert its effective efficacy.

The fibrotic tissue may be a liver, lung, heart, vascular system, articular or interstitial tissue, pancreas, skin, mouth, digestive tract, brain, breast, bone marrow, peritoneum, or kidney tissue in which a fibrotic condition has occurred, and preferably may be pulmonary fibrotic tissue, without being limited thereto.

According to another embodiment of the present invention, the present invention relates to a pharmaceutical composition for preventing, alleviating or treating fibrotic disease, comprising, as an active ingredient, a prodrug compound provided by the present invention.

According to another embodiment of the present invention, the present invention relates to a method for preventing, alleviating or treating fibrotic disease, comprising a step of administering a pharmaceutically effective amount of the prodrug compound of the present invention to a subject in need of administration.

In the present invention, the fibrotic disease may be, but is not limited to, scleroderma, atherosclerosis, cardiac fibrosis, organ transplant fibrosis, muscular fibrosis, pancreatic fibrosis, myelofibrosis, hepatic fibrosis, splenic fibrosis, pulmonary fibrosis, idiopathic pulmonary fibrosis, idiopathic interstitial fibrosis, diffuse interstitial fibrosis, interstitial lung disease, chronic interstitial lung disease, pneumoconiosis, silicosis, interstitial fibrosis, sarcoidosis, mediastinal fibrosis, cardiac fibrosis, atrial fibrosis, endocardial fibrosis, renal fibrosis, macular degeneration, keloid lesions, hypertrophic scars, nephrogenic systemic fibrosis, injection fibrosis, fibrotic complications of surgery, fibrotic chronic allograft vasculopathy, ischemic reperfusion injury-associated fibrosis, arthrofibrosis, Dupuytren's disease, fibrous proliferative lesions of the oral cavity, fibrotic intestinal stenosis, glial scar formation, leptomeningeal fibrosis, fibrosis due to radiation exposure, fibrosis due to mammary cystic rupture, myelofibrosis, retroperitoneal fibrosis, or progressive massive fibrosis, preferably idiopathic pulmonary fibrosis.

### Mode for Invention

Hereinafter, the present invention will be described in more detail through examples. These examples are only intended to explain the present invention in more detail, and it will be apparent to those skilled in the art that the scope of the present invention according to the subject matter of the present invention is not limited by these examples.

### Examples

### [Example 1] Synthesis of FAAP

A compound (FAAP) represented by Formula 8 below was synthesized according to Reaction Scheme 3 below.

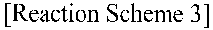

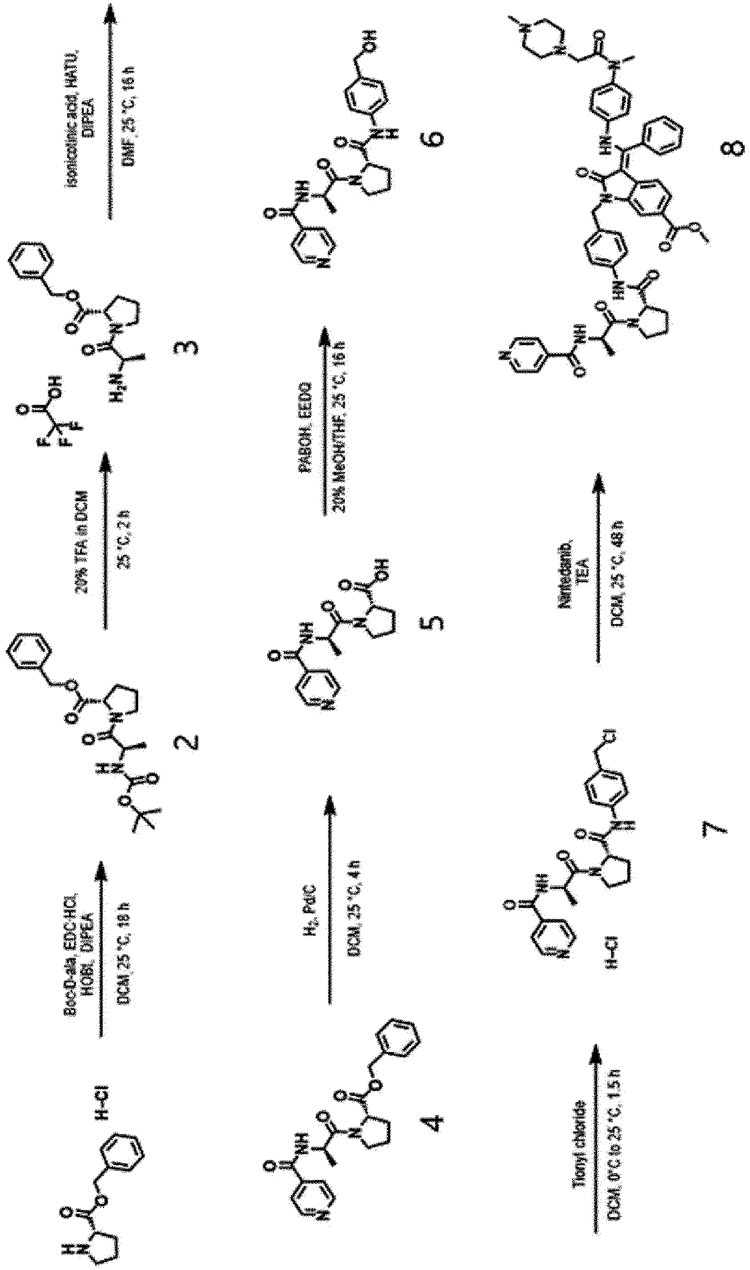

### 1. Production of (S)-benzyl 1-((R)-2-((tert-butoxycarbonyl)amino)propanoyl)pyrrolidone-2-carboxylate (Formula 2)

(tert-butoxycarbonyl)-D-alanine (3.62 g, 20.7 mmol), EDCHCl (4.76 g, 24.84 mmol), HOBt (3.36 g, 24.84 mmol), and DIPEA (14.4 ml, 82.8 mmol) were added to a solution of benzyl L-prolinate hydrochloride (5 g, 20.7 mmol) in 200 mL of DCM. The mixture was stirred at 25°C for 18 hours. The mixture was extracted with 0.1 M HCl (100 mL x 3), and the organic layer was extracted again with brine (50 mL x 3). The extract was dried over Na₂SO₄ and concentrated, and the residue was purified by flash chromatography (FC) (ethyl acetate (EtOAc) / hexane = 4/6) to obtain 4.78 g of clear oil 2 (yield: 63.7%).

¹H NMR (400 MHz, CD₃OD) δ 7.41-7.29 (m, 5H), 5.22 (s, 1H), 5.14 (s, 1H), 4.49-4.42 (m, 1H), 4.09-4.05 (m, 1H), 3.84-3.80 (m, 1H), 3.63-3.49 (m, 1H), 2.26-1.92 (m, 4H), 1.46-1.40 (m, 9H), 1.28-1.26 (d, *J* = 8 Hz, 2H), 1.06-1.04 (d, *J* = 8 Hz, 1H). MS (ESI) *m*/*z* 377.2 (M+H)⁺

### 2. Production of (S)-benzyl 1-((R)-2-aminopropanoyl)pyrrolidone-2-carboxylate 2,2,2-trifluoroacetate (Formula 3)

A solution of 1 (4.78 g, 13.18 mmol) in 100 mL of 20% TFA/DCM was stirred at room temperature for 2 hours. The mixture was concentrated and H₂O (50 mL) was added thereto, followed by freeze drying to obtain 5 g of pale yellow oil 3 (yield 99%).

¹H NMR (400 MHz, CD₃OD) δ 7.40-7.32 (m, 5H), 5.23-5.12 (m, 2H), 4.53-4.50 (m, 1H), 4.32-4.26 (m, 1H), 3.78-3.74 (m, 1H), 3.63-3.56 (m, 1H), 2.31-1.98 (m, 4H), 1.50-1.22 (m, 3H). ¹H NMR (400 MHz, CD₃OD) δ 7.40-7.32 (m, 5H), 5.23-5.12 (m, 2H), 4.53-4.50 (m, 1H), 4.32-4.26 (m, 1H), 3.78-3.74 (m, 1H), 3.63-3.56 (m, 1H), 2.31-1.98 (m, 4H), 1.50-1.22 (m, 3H). MS (ESI) *m*/*z* 277.1 (M+H)⁺

### 3. Preparation of (S)-benzyl 1-((R)-2-(isonicotinamido)propanoyl)pyrrolidone-2-carboxylate (Formula 4)

Isonicotinic acid (0.629 g, 5.11 mmol), HATU (2.9 g, 7.665 mmol), and DIPEA (2.67 mL, 15.33 mmol) were added to a solution of 2 (1.995 g, 5.11 mmol) in 50 mL of DMF. The mixture was stirred at room temperature for 16 hours. After concentrating the reaction mixture, the concentrate was diluted with 50 mL of DCM and then extracted with 0.1 M HCl (50 mL x 3), and the organic layer was extracted again with brine (50 mL x 3). The organic layer was dried over anhydrous sodium sulfate (Na₂SO₄) and filtered. The filtrate was concentrated, and the residue was purified by flash chromatography (FC) (methanol/dichloromethane = 1/9) to obtain 1.7 g of white foamy solid 4 (yield: 87%).

¹H NMR (400 MHz, CDCl₃) δ 8.72-8.69 (m, 2H), 7.91-7.89 (d, *J* = 8 Hz, 1H), 7.71-7.67 (m, 2H), 7.3-7.21 (m, 5H), 5.22-5.11 (m, 2H), 5.04-4.93 (m, 1H), 4.67-4.54 (m, 1H), 4.12-3.56 (m, 2H), 2.30-2.04 (m, 4H), 1.49-1.24 (m, 3H). MS (ESI) *m*/*z* 382.1 (M+H)⁺

### 4. Production of (S)-1-((R)-2-(isonicotinamido)propanoyl)pyrrolidine-2-carboxylic acid (Formula 5)

10 wt% Pd/C (470.4 mg, 0.46 mmol) was added to a solution of 3 (1.7 g, 4.46 mmol) in 100 mL of 20% MeOH/DCM, and the mixture was stirred at room temperature for 4 hours while bubbling hydrogen gas. The mixture was filtered through a Celite filter, and the filtrate was concentrated to obtain 1.13 g of white foamy solid 5 (yield: 87%).

¹H NMR (400 MHz, CD₃OD) δ 8.69-8.67 (m, 2H), 7.84-7.80 (m, 2H), 4.39-4.33 (m, 1H), 3.77-3.32 (m, 2H), 3.07-2.98 (m, 1H), 2.60-1.81 (m, 4H), 1.46-1.29 (m, 3H). MS (ESI) *m*/*z* 292.2 (M+H)⁺

### 5. Production of N-((R)-1-((S)-2-((4-(hydroxymethyl)phenyl)carbamoyl)pyrrolidin-1-yl)-1-oxopropan-2-yl)isonicotinamide (Formula 6)

EEDQ (445.7 mg, 1.80 mmol) and PABOH (221.9 mg, 1.80 mmol) were added to a solution of 4 (500 mg, 1.72 mmol) in 50 mL of 20% MeOH/THF. The mixture was stirred at 25°C for 16 hours. After concentrating the reaction mixture, the residue was purified by flash chromatography (FC) (methanol/dichloromethane = 1/9) to obtain 260 mg of beige foamy solid 6 (yield: 38%).

¹H NMR (400 MHz, CD₃OD) δ 8.69-8.67 (m, 2H), 7.84-7.80 (m, 2H), 7.64-7.57 (m, 2H), 7.34-7.27 (m, 2H), 4.59-4.55 (m, 3H), 4.03-3.64 (m, 2H), 3.32-3.30 (m, 1H), 2.34-2.06 (m, 4H), 1.48-1.33 (m, 3H). MS (ESI) *m*/*z* 397.2 (M+H)⁺

### 6. Production of N-((R)-1-((S)-2-((4-(chloromethyl)phenyl)carbamoyl)pyrrolidin-1-yl)-1-oxopropan-2-yl)isonicotinamide hydrochloride (Formula 7)

Thionyl chloride (331.6 µL, 4.57 mmol) was added to a solution of 5 (181 mg, 0.457 mmol) in 10 mL of DCM. The mixture was stirred at 0°C for 30 minutes, and then stirred at room temperature for 1 hour. After concentrating the reaction mixture, 10 mL of DCM was added and the mixture was concentrated again. This concentration process was repeated twice before proceeding with the next reaction.

¹H NMR (400 MHz, CD₃OD) δ 9.70-9.68 (m, 2H), 8.73-8.69 (m, 2H), 7.64-7.57 (m, 2H), 7.34-7.27 (m, 2H), 4.47-4.43 (m, 3H), 3.31-2.92 (m, 2H), 2.61-2.59 (m, 1H), 1.96-1.68 (m, 4H), 1.41-1.26 (m, 3H). MS (ESI) *m*/*z* 415.4 (M+H)⁺

### 7. Production of (Z)-methyl 1-(4-((S)-1-((R)-2-(isonicotinamido)propanoyl)pyrrolidine-2-carboxamido)benzyl)-3-(((4-(N-methyl-2-(4-methylpiperazin-1-yl)acetamido)phenyl)amino)phenyl)methylene)-2-oxoindoline-6-carboxylate (Chemical Formula 8)

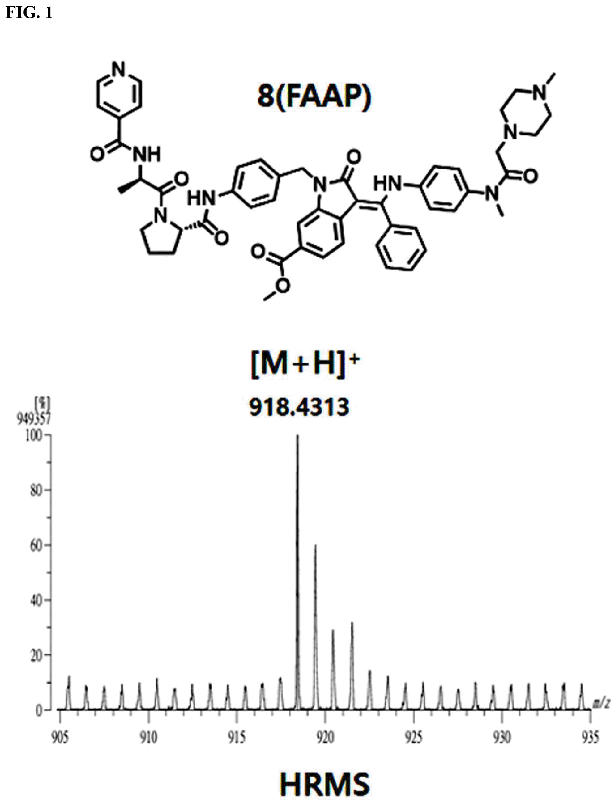

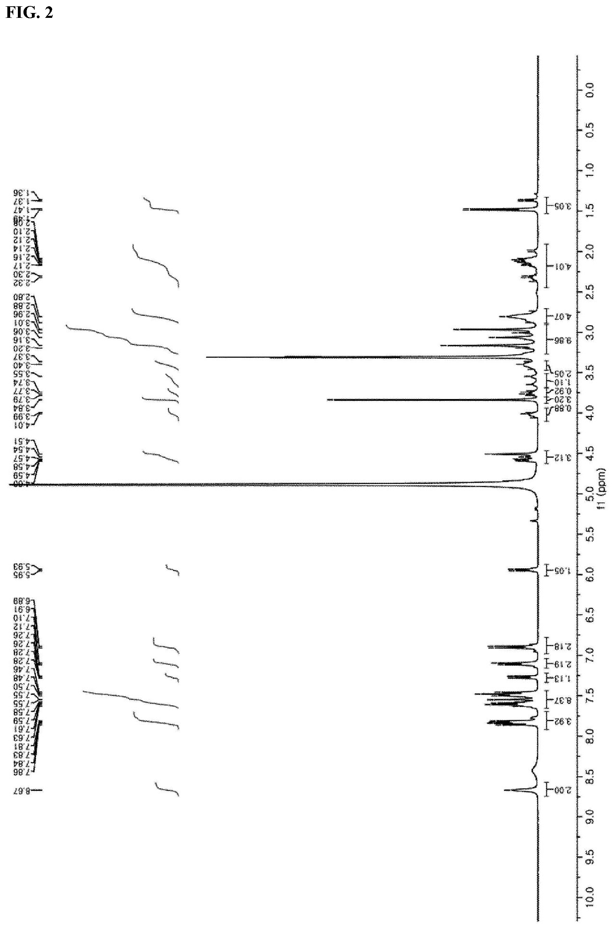

Compound 7 was dissolved in 10 mL of DCM, and nintedanib (197 mg, 0.3656 mmol) and TEA (239 µL, 1.371 mmol) were added to the solution. The mixture was stirred at room temperature for 48 hours. After concentrating the reaction mixture, the residue was purified by a Biotage^{®} Sfär KP-Amino D Duo 50 µm 5 g column (methanol/dichloromethane = 1/19), further purified by HPLC, and then freeze-dried to obtain 68.1 mg of yellow solid 8 (yield: 16%) (FIGS. 1 and 2).

¹H NMR (400 MHz, CD₃OD) δ 8.67 (s, 2H), 7.86-7.81 (m, 4H), 7.63-7.46 (m, 8H), 7.28-7.26 (dd, J = 8.2, 1.6 Hz, 1H), 7.12-7.10 (d, J = 8.4 Hz, 2H), 6.91-6.89 (d, J = 8.4 Hz, 2H), 5.95-5.93 (m, 1H), 4.60-4.51 (m, 3H), 4.06-3.99 (m, 1H), 3.84 (s, 3H), 3.79-3.73 (m, 1H), 3.65-3.55 (m, 1H), 3.40 (m, 2H), 3.20-2.96 (m, 10H), 2.88-2.74 (m, 4H), 2.37-1.98 (m, 4H), 1.49-1.36 (m, 3H). HRMS (FAB) *m*/*z* 918.4313 (M+H)⁺

### 8. Purity analysis

As a result of analyzing the purity by identifying the m/z value of the FAAP compound through HPLC and LC/MS analysis, it could be confirmed that FAAP was contained in the product with a purity of about 60% or more.

### [Examples 2 to 52] Synthesis of Prodrugs

The prodrug compounds shown in Table 1 below were synthesized by adding the drugs shown in Table 1 below instead of nintedanib in step 7 of Example 1 above.

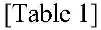

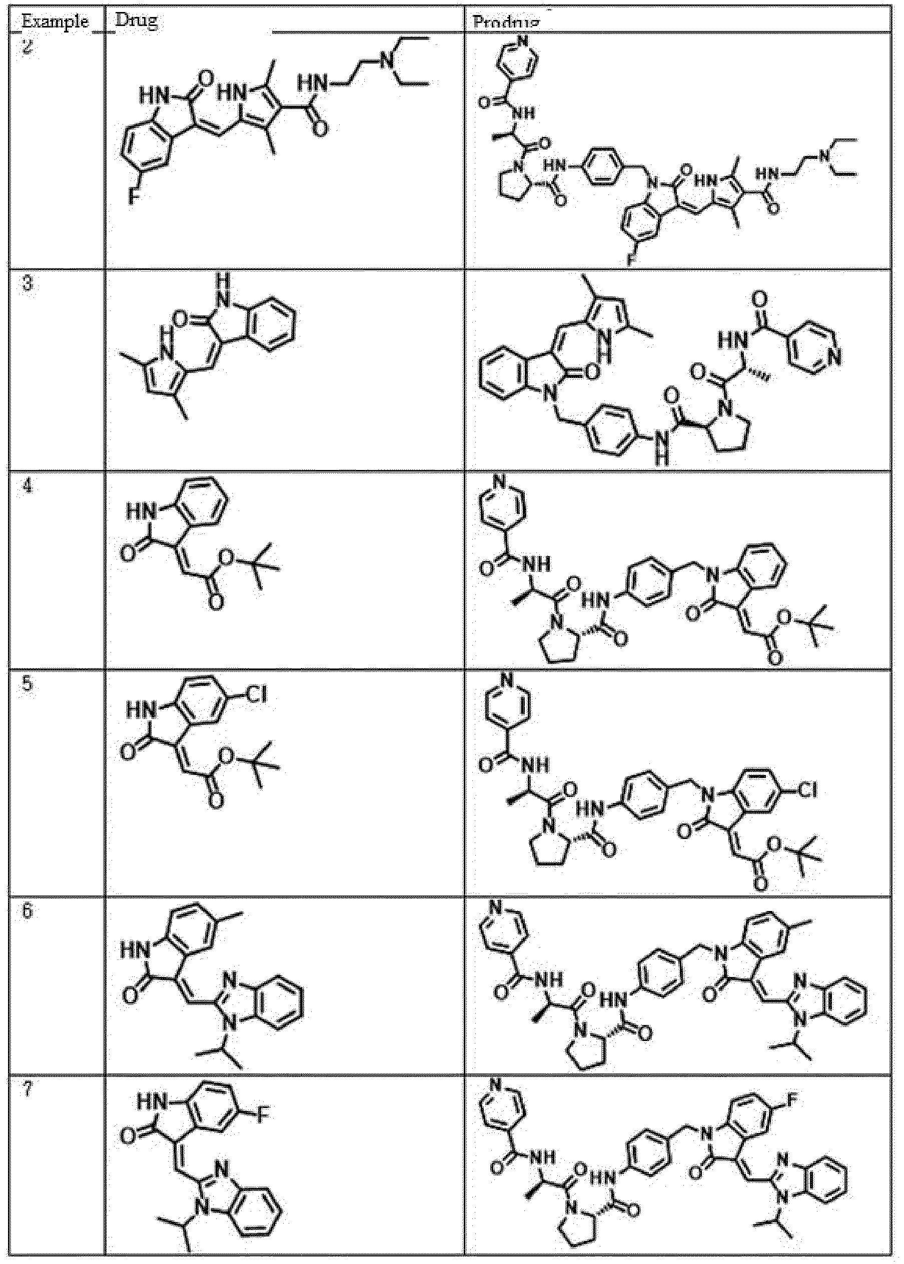

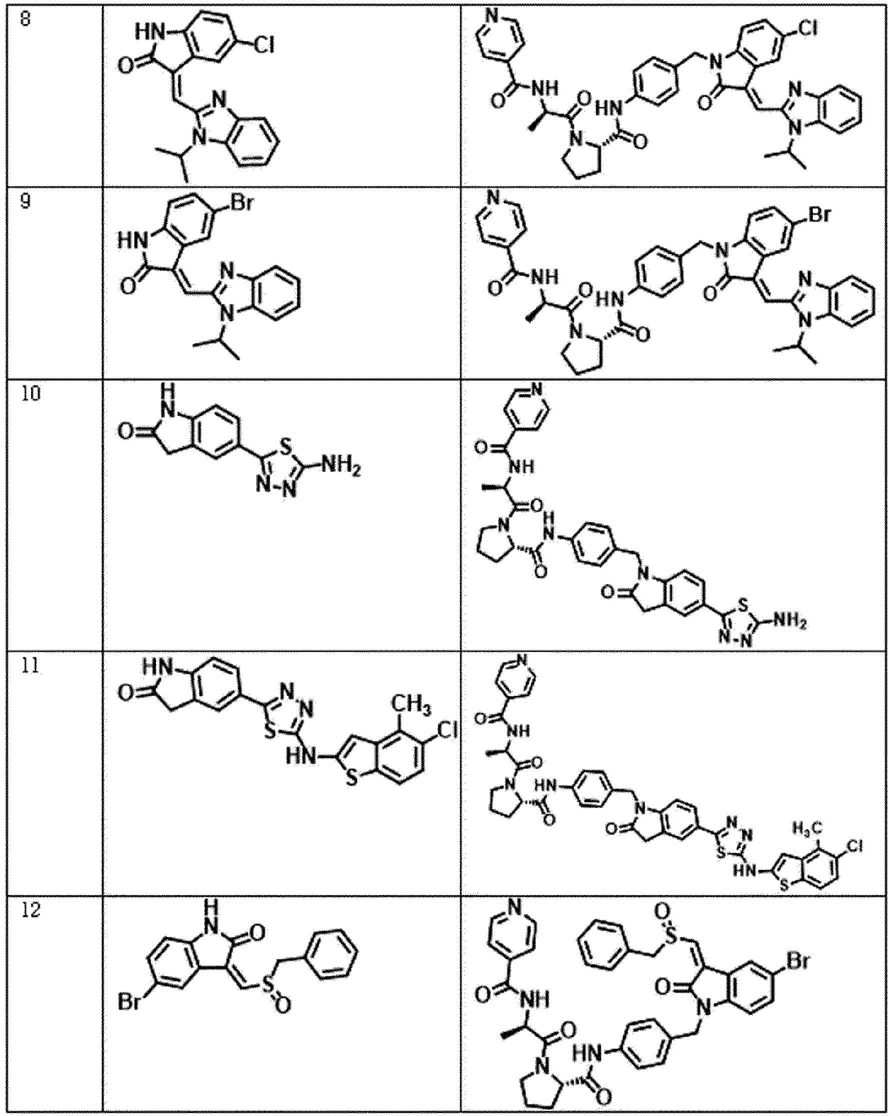

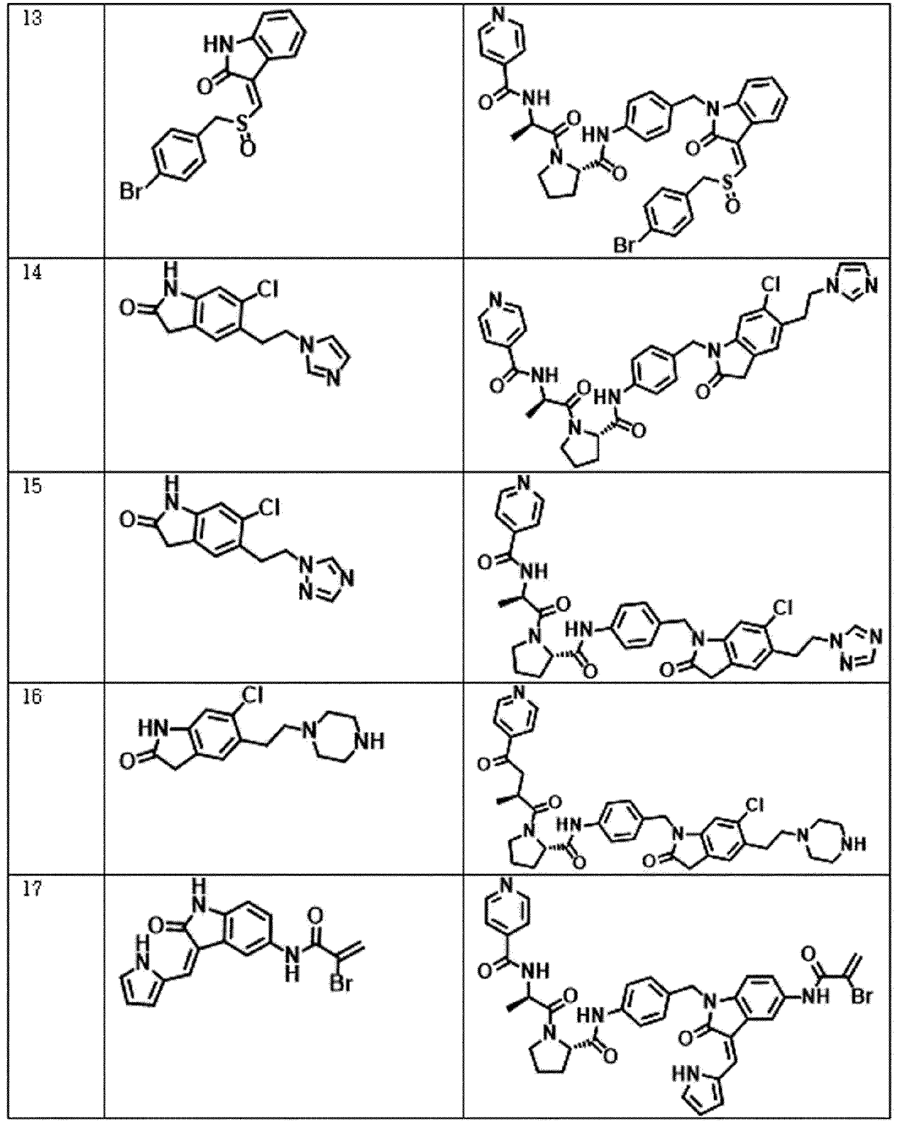

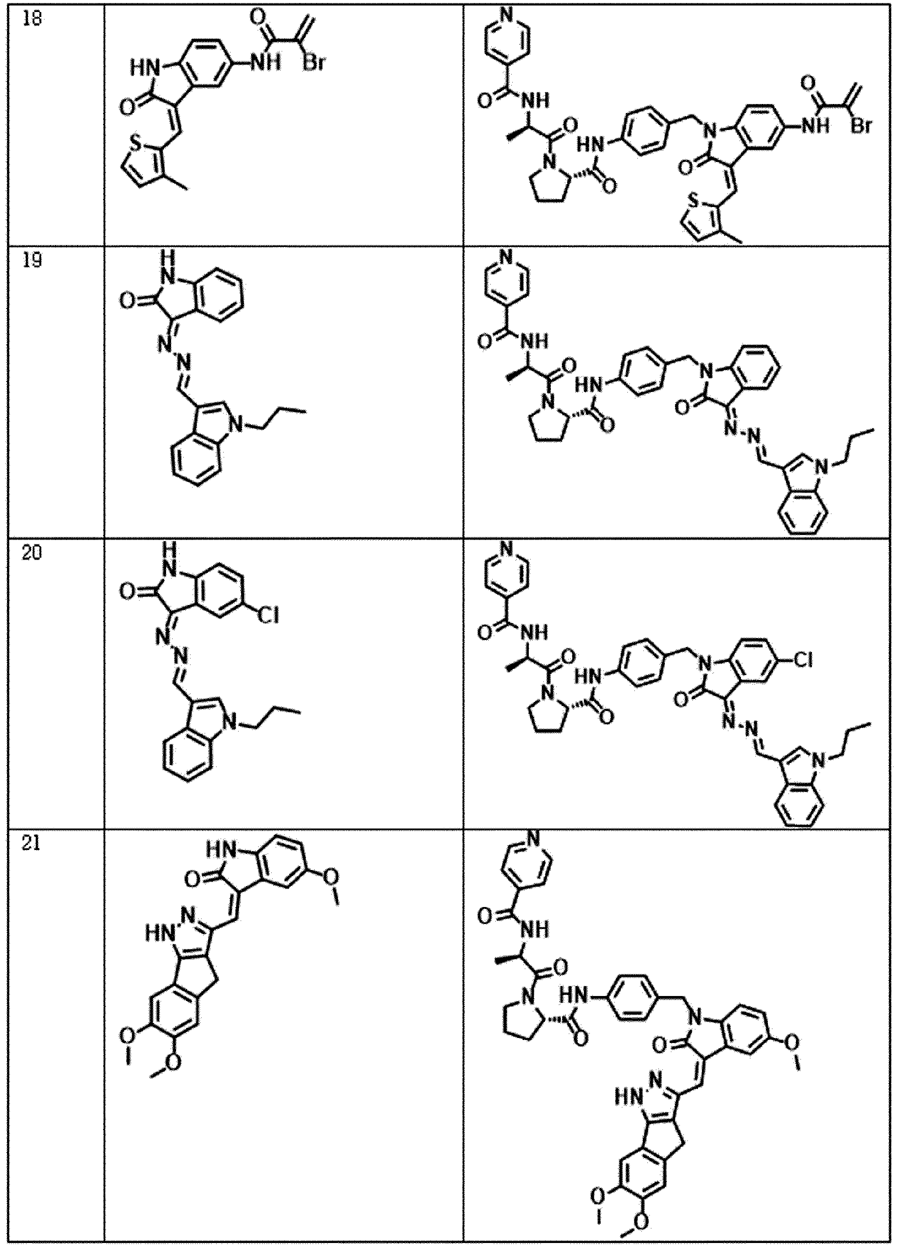

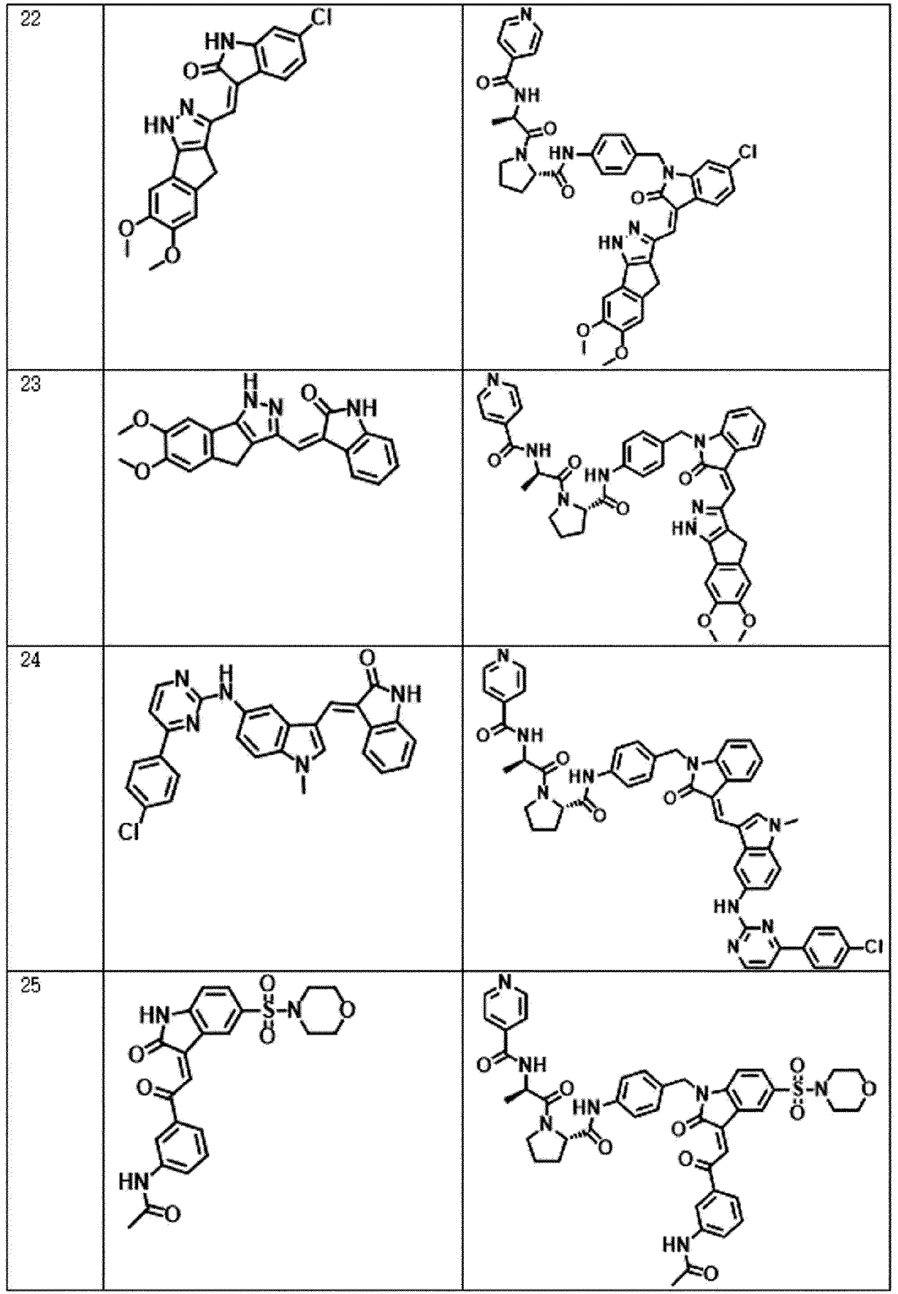

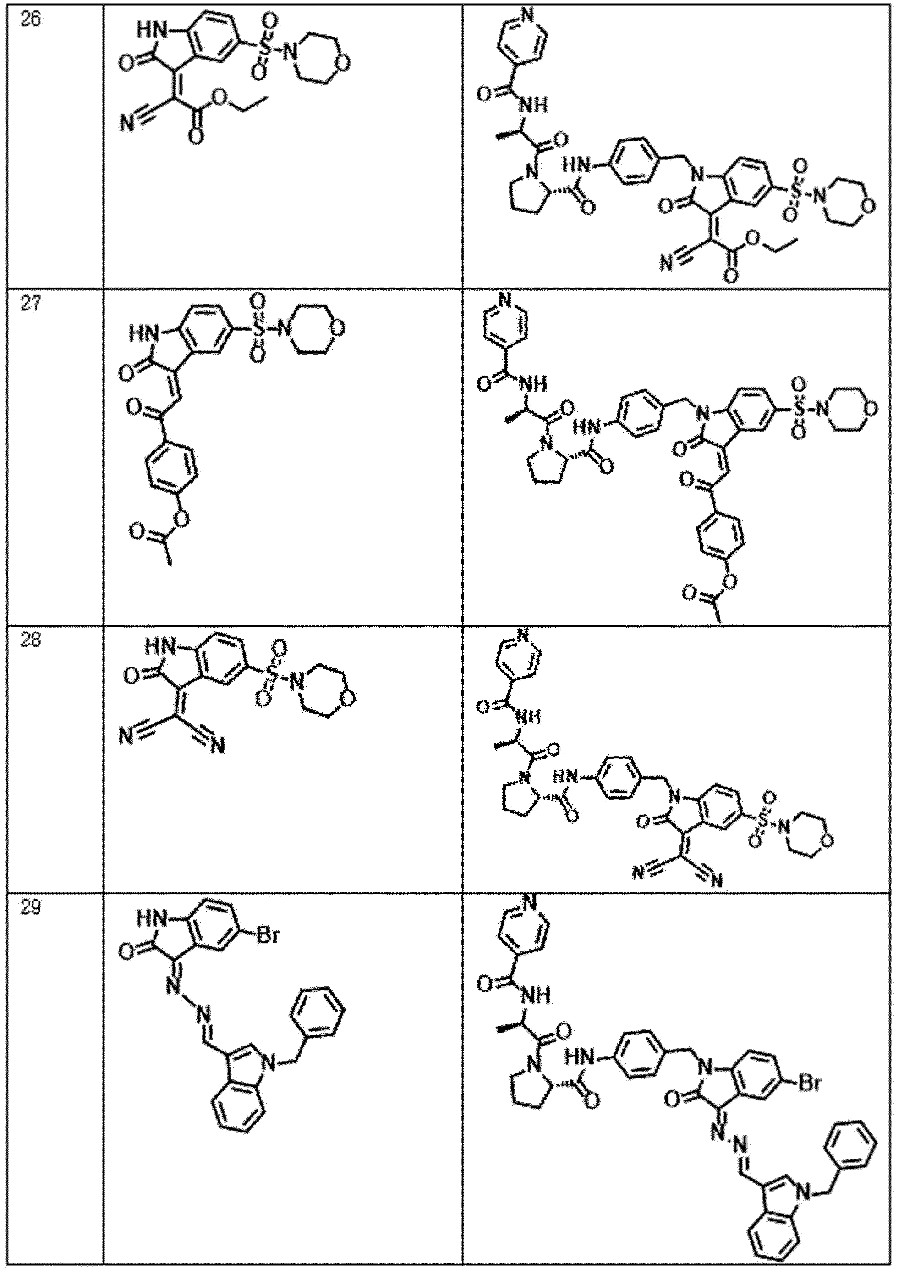

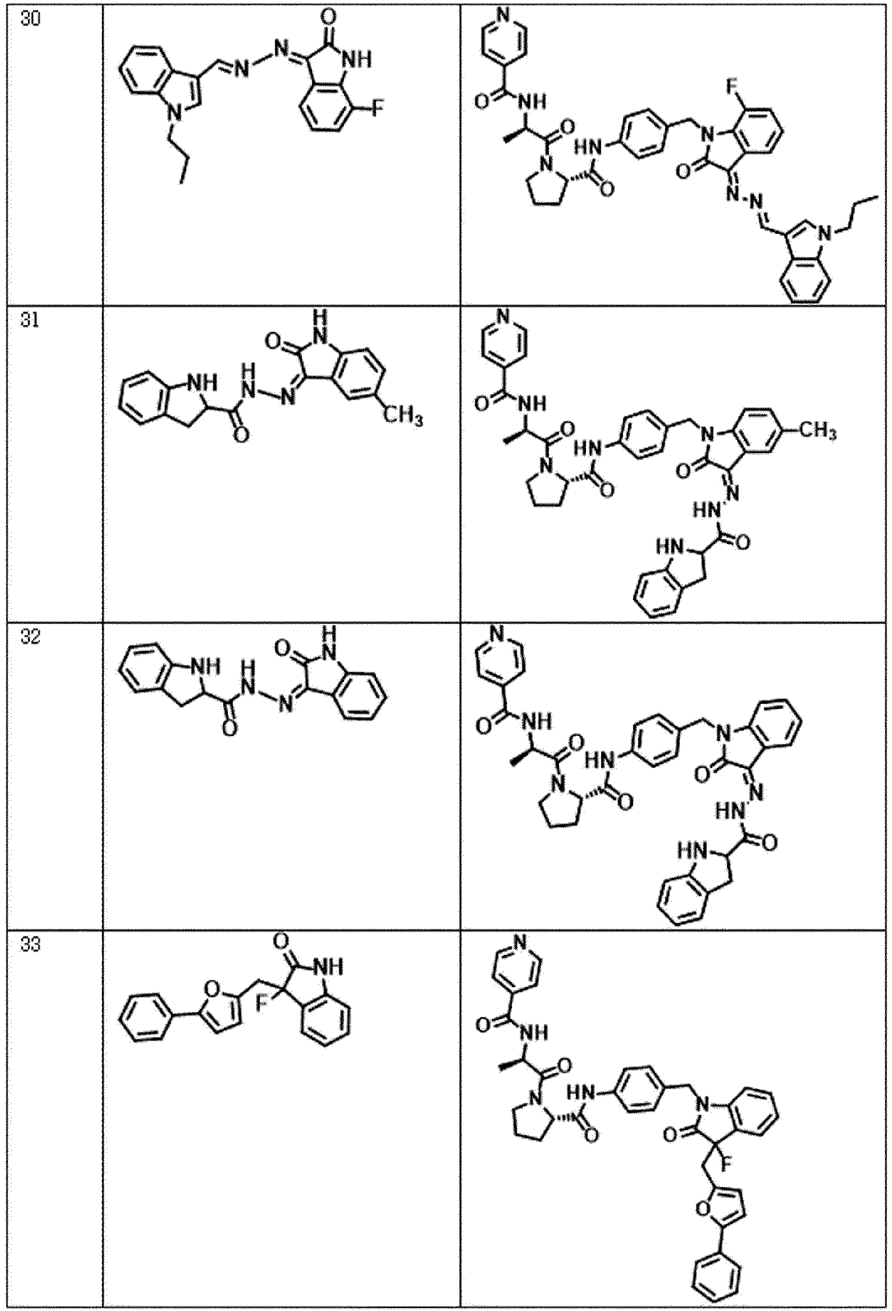

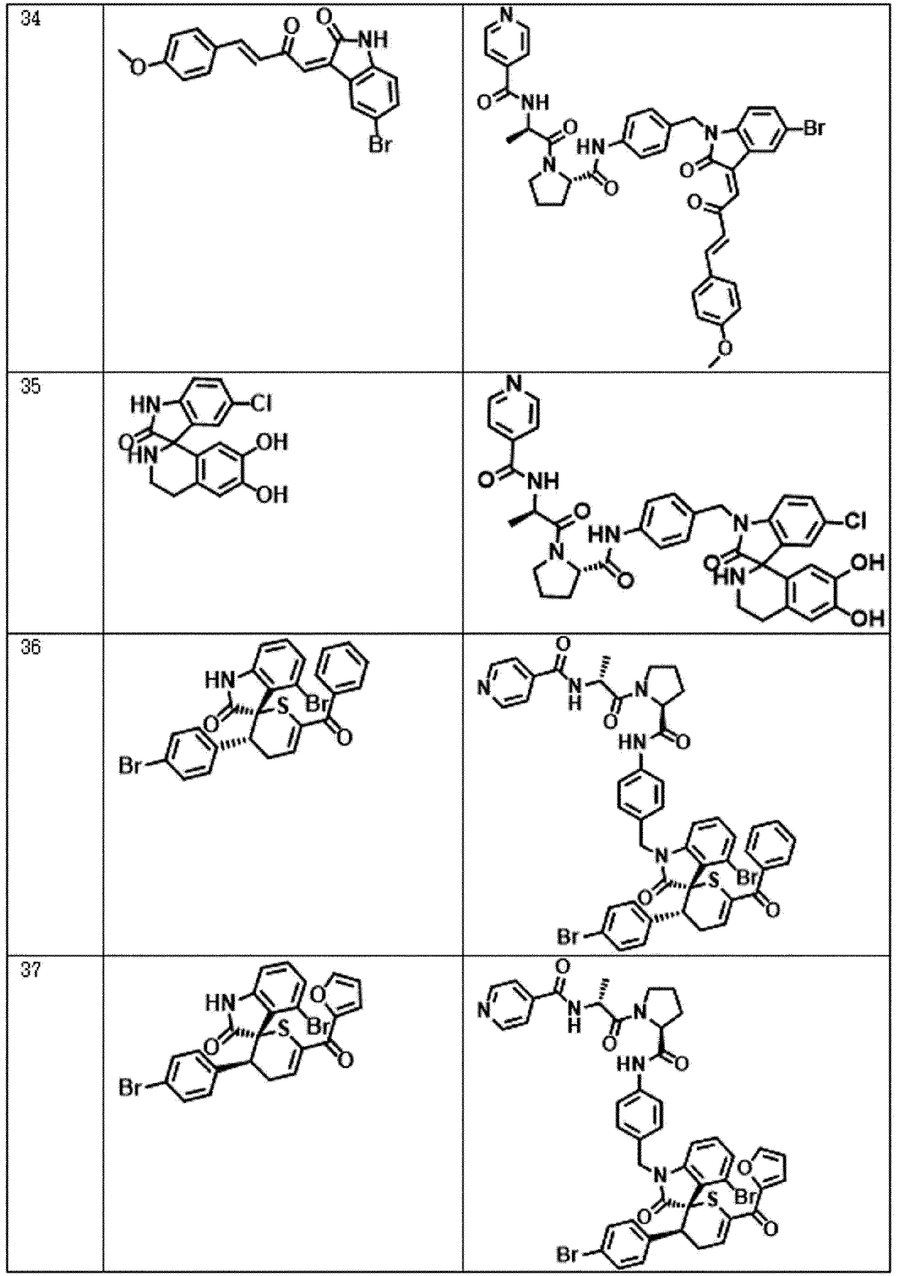

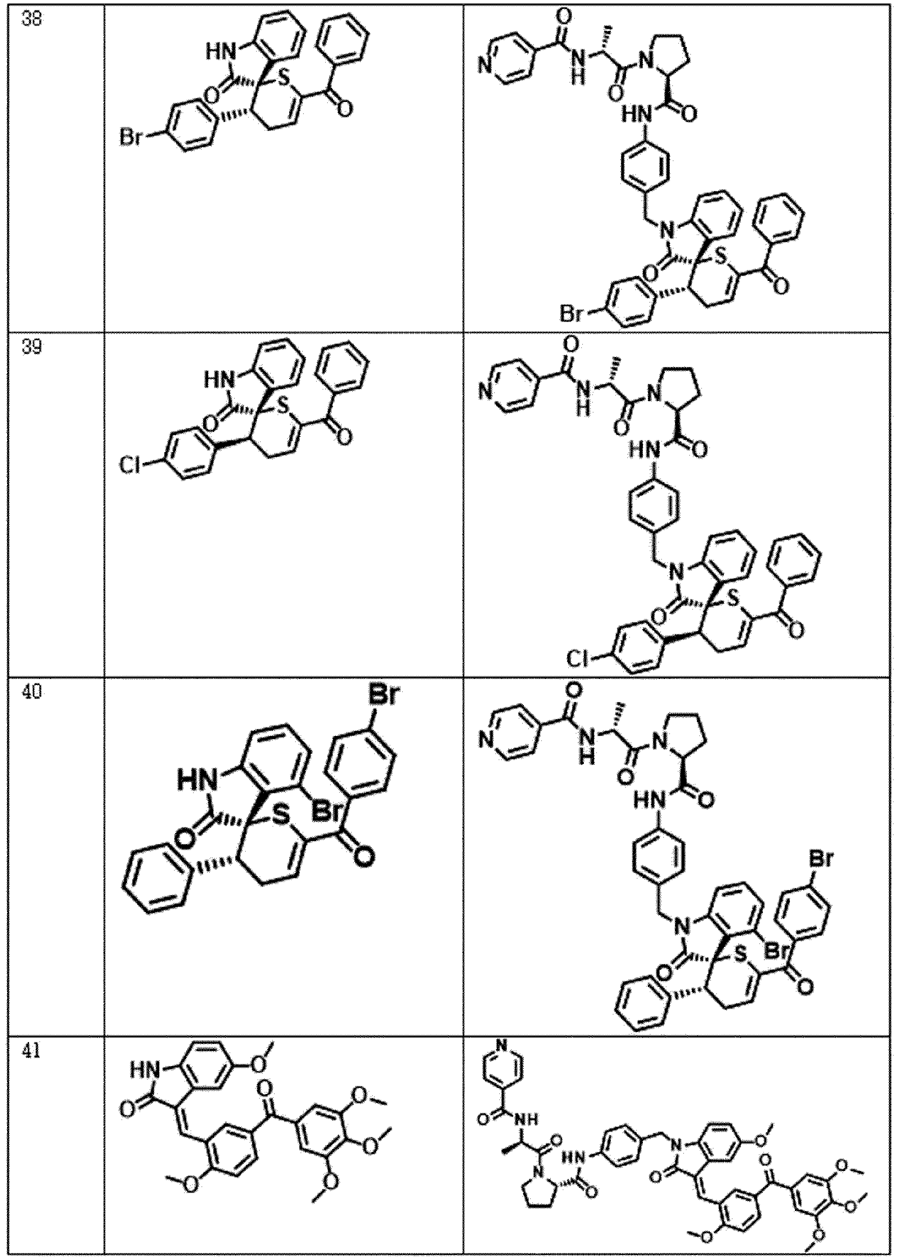

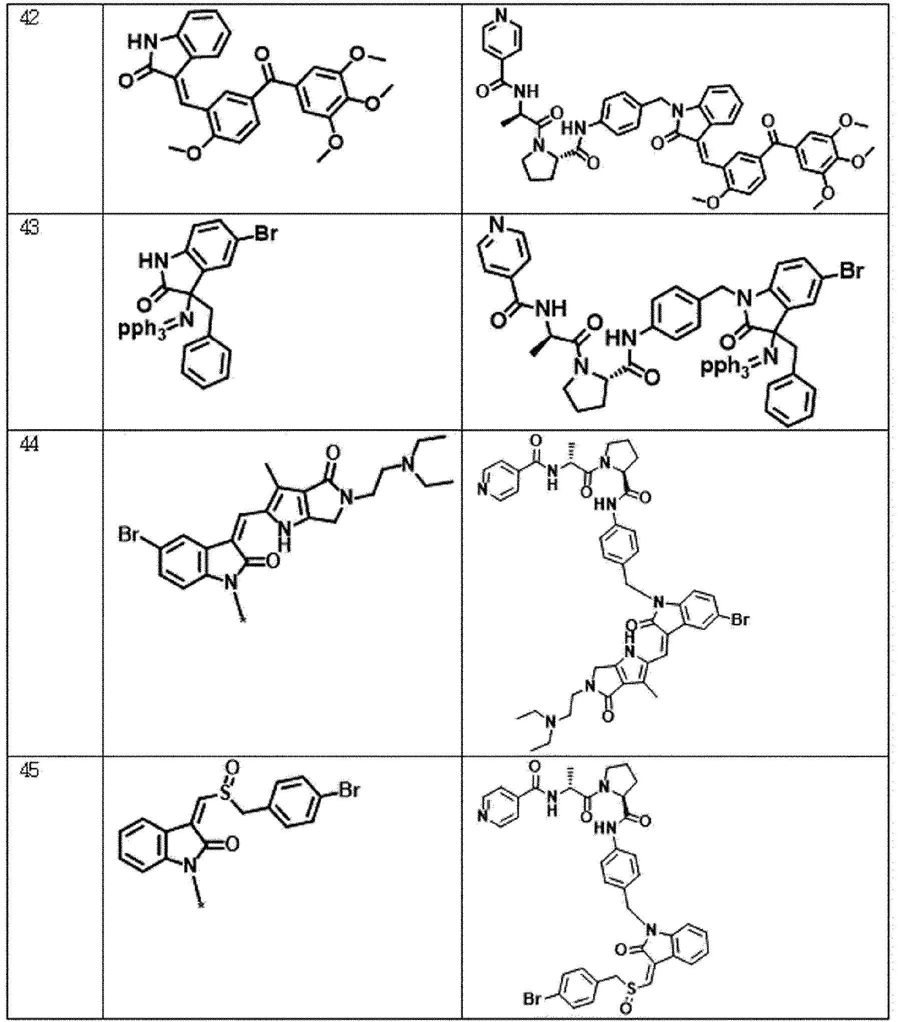

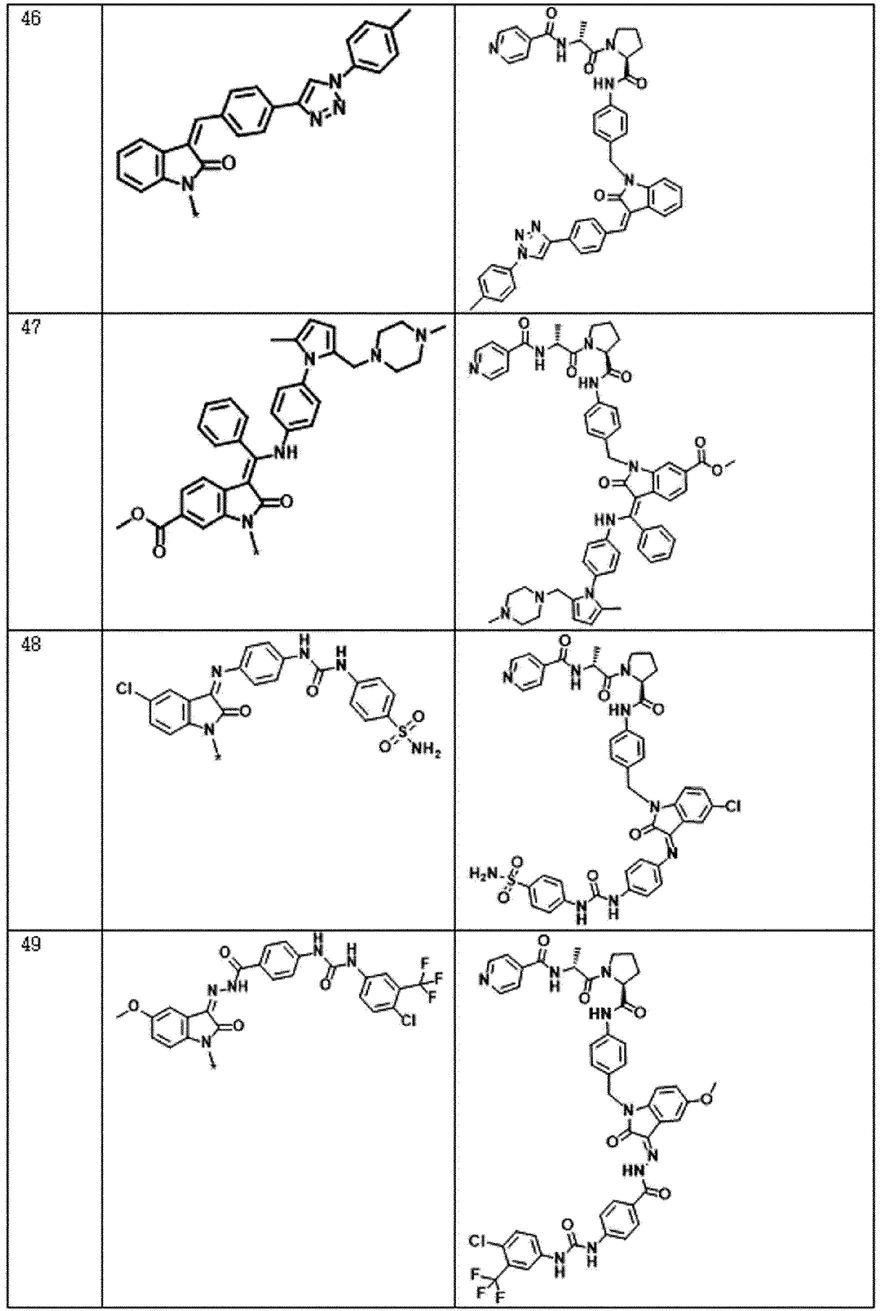

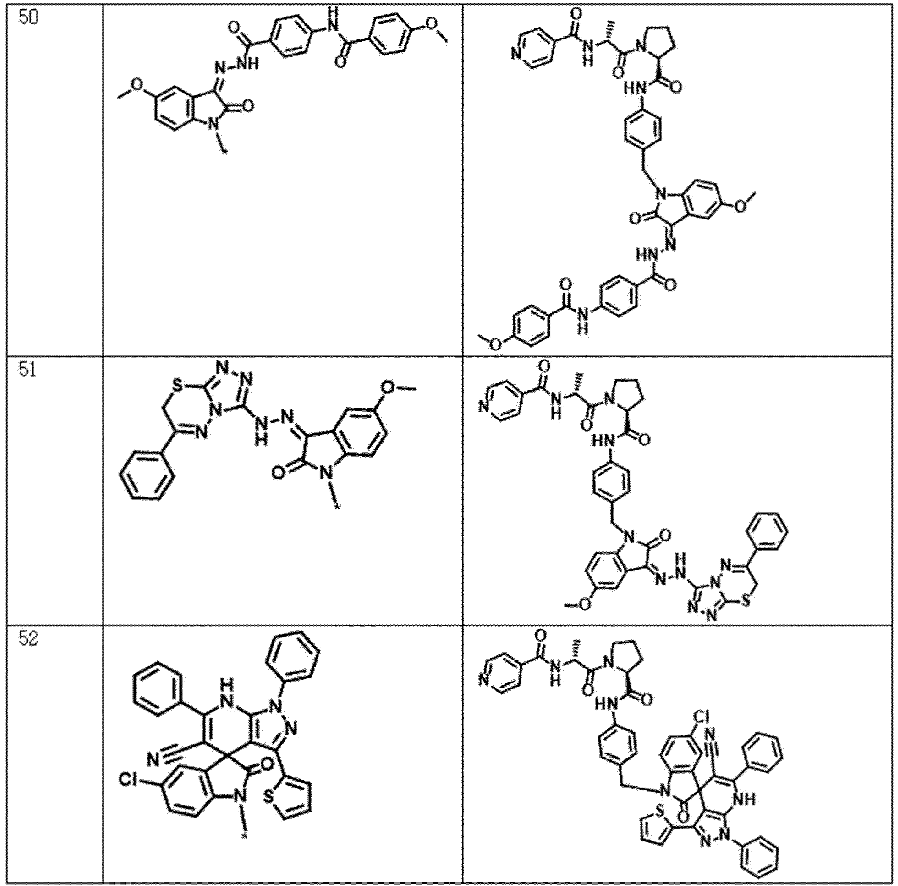

### [Experimental Example 1] Evaluation of Whether Substrate Is Degraded by FAP

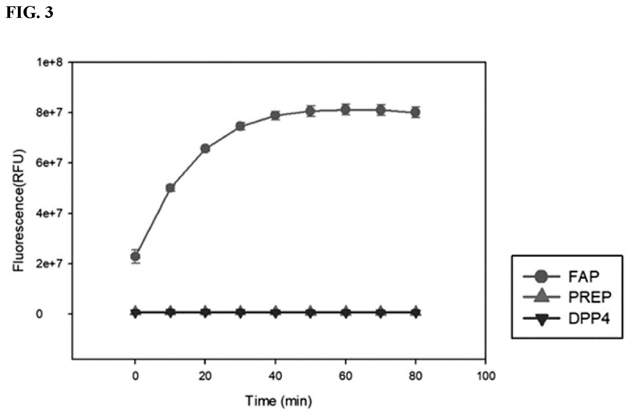

To evaluate whether the FAP substrate according to the present invention is specifically recognized and cleaved only by FAP, the following experiment was conducted. 2.5 µM of an AMC-conjugated isonicotinoyl-D-Ala-Pro moiety represented by Formula 9 below as a substrate, 2.5 µg/mL of FAP, and <1% (v/v) of DMSO were added and incubated in buffer (0.01 M Tris-HCl, pH 7.4, 0.01 M MgCl₂, 0.05% Tween-20, distilled water) at 37°C. As controls, PREP and DPP4, which are peptidases similar to FAP, were added. Fluorescence intensity as a function of incubation time was measured using a microplate instrument (SYNERGY H1, BioTek), and the results are shown in FIG. 3.

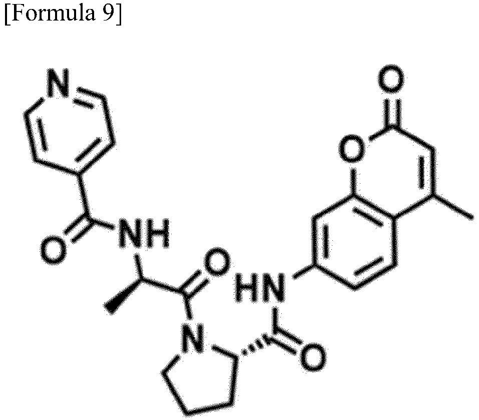

As shown in FIG. 3, it could be confirmed that the substrate according to the present invention was specifically cleaved only by the FAP enzyme and fluorescence was detected, but the substrate was not cleaved by PREP or DPP4, which has a structure similar to FAP.

### [Experimental Example 2] Evaluation of Whether Substrate Is Degraded by FAP

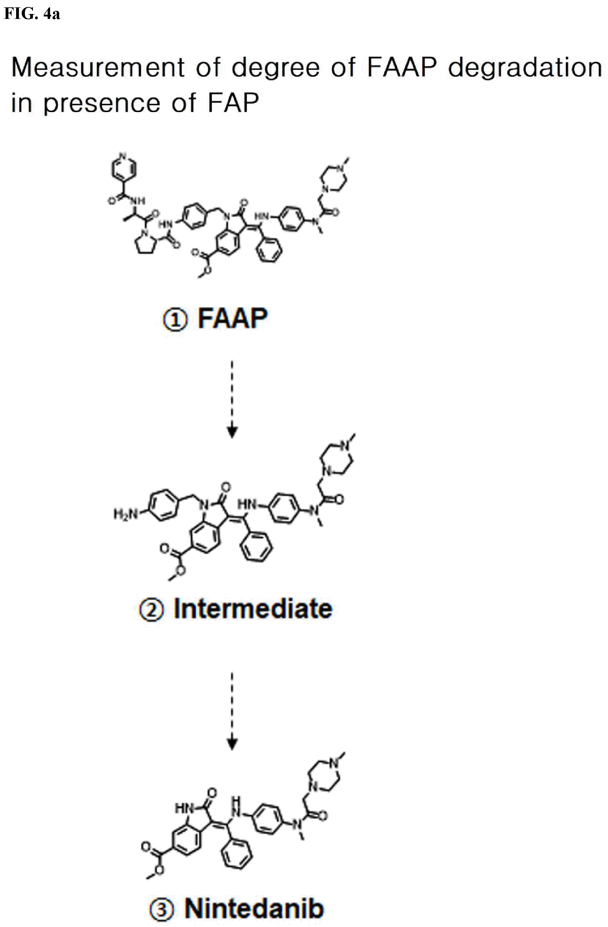

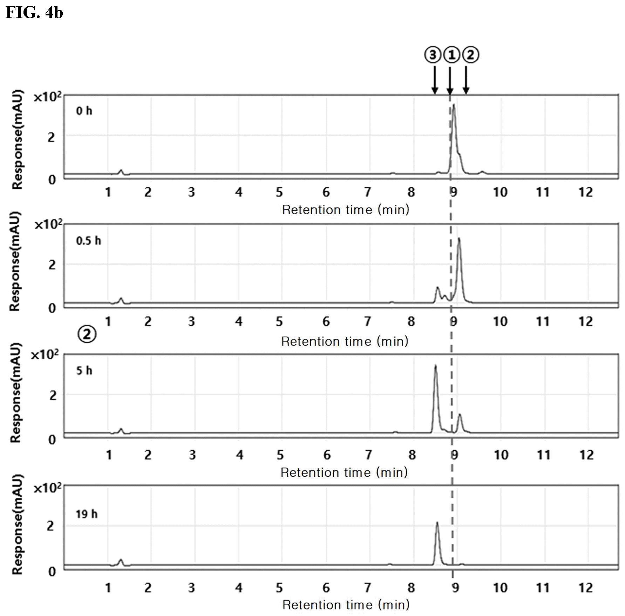

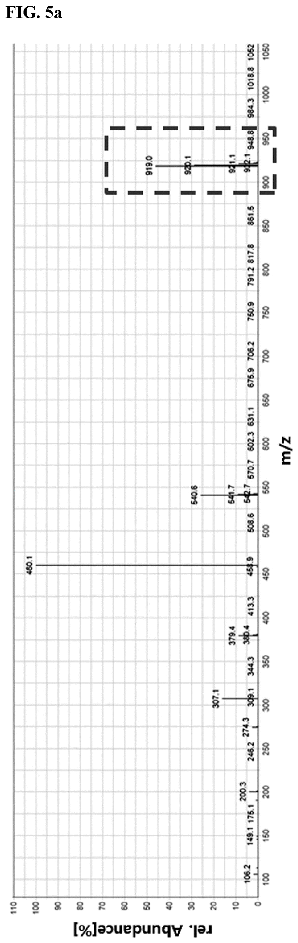

To evaluate the degree to which FAAP synthesized in Example 1 above is activated by FAP, 100 µM of FAAP and 4.45 µg/mL of FAP were incubated in a buffer (0.01 M Tris-HCl, pH 7.4, 0.01 M MgCl₂, 0.05% Tween-20, distilled water) and <2.5% (v/v) of DMSO at 37°C. Subsequently, samples were collected at different time points, and whether nintenanib would be released by degradation of FAAP was analyzed by HPLC and LC/MS using the instrument's mass detector and UV detector (390 nm). The results are shown in FIGS. 4a and 4b and FIGS. 5a to 5c. Here, the conditions for analysis using the HPLC and LC-MS systems (Agilent) are as follows:
Analysis column: X Terra MS C18 column, 25 Å, 3.5 µm, 2.1 mm x 100 mm;
HPLC and LC-MS conditions: 0-2 min, 20% A/B; 2-15 min, 20-90% A/B; 15-20 min, 90% A/B; 0.25 ml/min flow rate;
As elution buffer, acetonitrile (solvent A) and water (solvent B) are mixed with 0.1% formic acid (v/v).

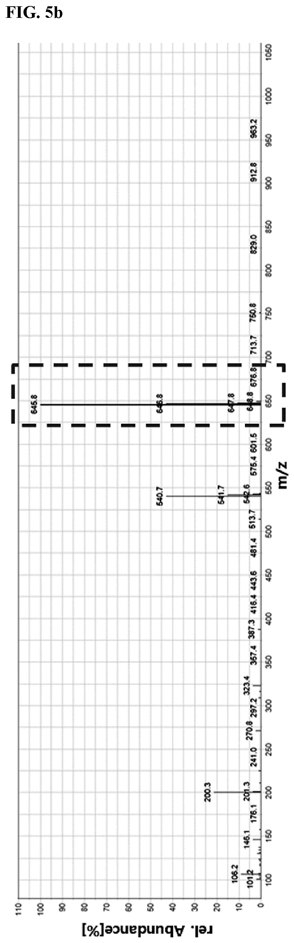

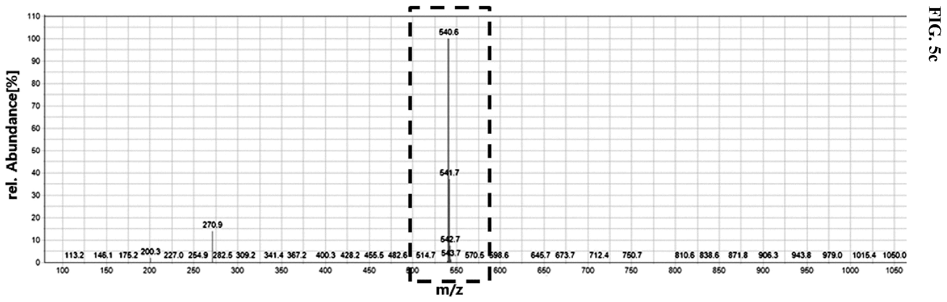

As shown in FIGS. 4 and 5, it was confirmed through LC/MS analysis that the [M + H]⁺ value of the FAAP compound (①) was about 919, the [M + H]⁺ value of the precursor (②) generated by degradation in the presence of FAP was about 645, and the [M + H]⁺ value of nintedanib (③) was 540. In addition, it could be confirmed that 40% of FAAP was degraded within 1.5 hours, 80% of FAAP was degraded within 5 hours, and FAAP was completely degraded into nintedanib at a subsequent time point, so that the prodrug was activated. Meanwhile, it could be confirmed that, in the absence of FAP, the compound remained very stable for a long period.

### [Experimental Example 3] Evaluation of Binding Affinity of FAAP to Tyrosine Kinase

Nintedanib, a drug contained in the FAAP of the present invention, is known to bind to three types of tyrosine kinases (VEGFR2, FGFR1, and PDGFRα). Through molecular docking simulation (AutoDock Vina), docking simulation was performed between nintedanib and the FAAP structure in the region corresponding to the pocket of the receptor. However, the types of receptors used in the experiment were VEGFR-2 crystal (PDB ID: 3C7Q) and PDGFRα (PDB ID: 6JOL). The binding affinity and binding site of nintedanib in the FAAP structure to tyrosine kinase were analyzed, and the results are shown in Table 2 below.

**[Table 2]**

| Compound | VEGFR2 | PDGFRα |
|---|---|---|
| Nintedanib | -9.3 | 8.2 |
| Example 1 (FAAP) | -8.066 | -6.805 |

As a result, as shown in Table 2, it could be confirmed that the binding affinity of FAAP according to the present invention to tyrosine kinase was lower than that of nintedanib alone.

### [Experimental Example 4] Evaluation of Binding Affinity of FAAP to Target Kinases

This experiment was conducted based on a competitive binding assay for quantitatively measuring the ability of the compound to compete with an immobilized, active site-directed ligand. This assay was performed by combining three components: a DNA-tagged kinase, a ligand immobilized on a bead, and the test compound. If the test compound binds to the kinase and directly or indirectly blocks the ATP site, the number of protein molecules (kinase) that bind to the ligand immobilized on the bead is reduced. If the test compound does not bind to the kinase, the tagged protein (kinase) can bind to the bead. Therefore, the degree of binding is evaluated based on the amount of fusion protein, bound to the bead, through quantitative PCR (qPCR). Kinase, liganded beads, and test compounds (nintedanib and FAAP were each diluted three-fold to the highest concentration of 10 mM and tested at a total of 11 points) were placed in a test plate and incubated with stirring at room temperature for 1 hour. Thereafter, the beads were resuspended in elution buffer (1x PBS, 0.05% Tween 20, 0.5 µM non-biotinylated affinity ligand) and then incubated with stirring at room temperature for 30 minutes, and the kinase concentration in the eluate was measured by qPCR. The binding affinity of FAAP to each kinase was compared with that of nintedanib, and the results are shown in Table 3 below.

**[Table 3]**

| Target | Binding affinity of FAAP compared to nintedanib |
|---|---|
| FGFR1 | 0.8-fold |
| FLT1 | 0.2-fold |
| PDGFRα | 0.47-fold |

As shown in Table 3 above, it could be confirmed that the binding affinity of FAAP according to the present invention to tyrosine kinases was lower than that of nintedanib. This suggests that the prodrug compound according to the present invention has a reduced likelihood of side effects as the binding thereto to tyrosine kinases expressed in normal organs is inhibited.

### [Experimental Example 5] Evaluation of FAAP Degradation Depending on Presence or Absence of Intracellular FAP

To evaluate whether FAAP is activated by FAP at the cellular level, a normal group with FAP and a group in which FAP was blocked using oncoFAP were treated with each of nintedanib and FAAP, and the uptake pattern was observed using a confocal microscope. Specifically, cells were seeded into confocal dishes at a density of 2 x 10⁵ cells/mL per well, incubated for 24 hours, and then subjected to drug treatment. For drug treatment, the normal group with FAP was treated with 2 µL (10 µM) of each of FAAP and nintedanib. The group in which FAP was blocked was first treated with 10 µL (1 mM) of oncoFAP, shaken from side to side, incubated for 30 minutes, and then treated with 2 µL (10 µM) of FAAP. After drug treatment, shaking was performed using a shaker at 165 rpm for 5 minutes. After incubation in an incubator for 15 minutes, 2 hours, 6 hours, and 10 hours, the cells were washed twice with warm DPBS. The cells were incubated with 1 ml of warm 4% PFA for 15 minutes, washed twice, then fixed by filling with DPBS to keep them from drying out, and then confocal images were taken. Nintedanib drug images were obtained using a Hoechst 33258 filter. The reason why it was possible to observe with a confocal microscope is that nintedanib has the property of exhibiting intrinsic fluorescence (emission: 482 nm, excitation: 390 nm), whereas FAAP does not exhibit fluorescence.

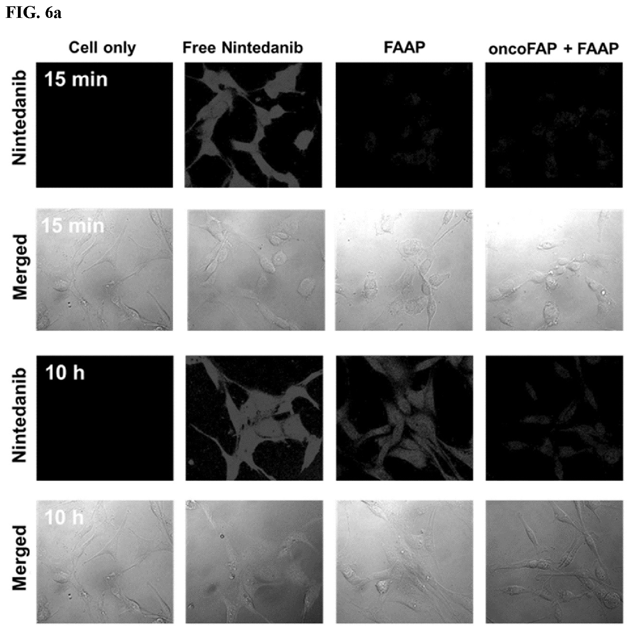

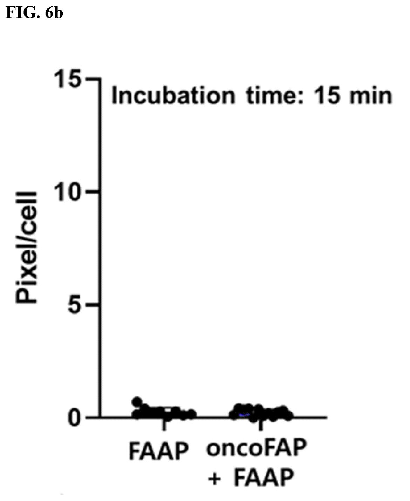

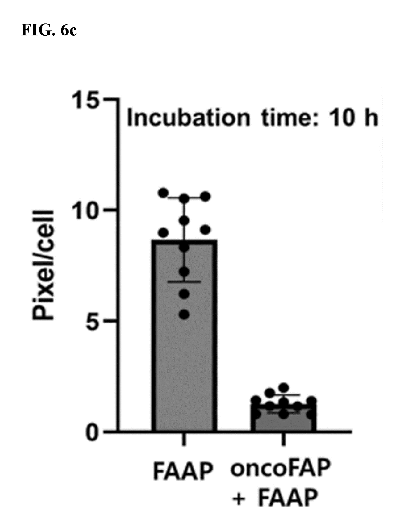

As shown in FIGS. 6a to 6c, it could be confirmed that nintedanib showed fluorescence starting 15 minutes after treatment, whereas FAAP did not show fluorescence until 10 hours after treatment of cells with FAAP, at which point the fluorescence intensity increased. In addition, it could be confirmed that, in the group treated with FAAP after blocking FAP with the FAP inhibitor OncoFAP, no fluorescence appeared even after 10 hours.

Through these experiments, it could be seen that the FAAP of the present invention is specifically activated into nintedanib by FAP at the cellular level.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

### Industrial Applicability

The present invention relates to a prodrug compound, which is activated by fibroblast activation protein (FAP) and may be applied as an anti-fibrotic agent, and the use thereof.

### [National Research and Development Project that supported this invention]

[Project Serial Number] 1465037025
[Project Number] HN22C0632000022
[Government Department] Ministry of Health and Welfare
[Project Management (Specialized) Agency] Korea Drug Development Foundation
[Research Project Name] National Drug Development Project (Ministry of Science and ICT, Ministry of Health and Welfare, and Ministry of Trade, Industry and Energy)
[Research Task Name] Research on Identification of Active Substances for Translocator Protein-Targeting Cancer Therapeutics
[Agency Carrying Out Project] Seoul National University R&DB Foundation
[Research Period] May 1, 2022 to April 30, 2025

### [National Research and Development Project that supported this invention]

[Project Serial Number] 1415180797
[Project Number] 20018522
[Government Department] Ministry of Trade, Industry and Energy
[Project Management (Specialized) Agency] Korea Planning & Evaluation Institute of Industrial Technology
[Research Project Name] Development of Advanced Production Technology for Vaccine Raw Materials
[Research Task Name] Development of Technology for Evaluation of mRNA Vaccine Product Quality and Efficacy
[Agency Carrying Out Project] Seoul National University
[Research Period] April 1, 2022 to December 31, 2025

### [National Research and Development Project that supported this invention]

[Project Serial Number] 1711168268
[Project Number] 2020R1C1C1009000
[Government Department] Ministry of Science and ICT
[Project Management (Specialized) Agency] National Research Foundation of Korea
[Research Project Name] Excellent Young Researcher Program
[Research Task Name] Development of Targeted Radionuclide-Photodynamic Combination Cancer Therapy Using Radioluminescent Liposomal Nanoplatform
[Agency Carrying Out Project] Seoul National University
[Research Period] March 1, 2020 to February 28, 2025

### [National Research and Development Project that supported this invention]

[Project Serial Number] 1711158495
[Project Number] 2021M2E8A1039564
[Government Department] Ministry of Science and ICT
[Project Management (Specialized) Agency] National Research Foundation of Korea
[Research Project Name] Research on Future Innovative Foundation Technologies for Radiation Utilization
[Research Task Name] Development of Radiation-Induced Photoimmunotherapy Using Antibody-Europium-Photosensitizer Complex
[Agency Carrying Out Project] Seoul National University
[Research Period] May 1, 2021 to December 31, 2024

[Project Serial Number] RS-2021-NR059015
[Project Number] 2021R1A2C2003301
[Government Department] Ministry of Science and ICT
[Project Management (Specialized) Agency] National Research Foundation of Korea
[Research Project Name] Mid-career Researcher Support Program (R1A2)
[Research Task Name] Development of pH- and Temperature-Sensitive Nanocomposite-Based Nanocarriers That Enhance Loading Capacity, Tumor Selectivity, and Retention of Mitochondrial-Targeted Tumor Therapeutic Agents
[Agency Carrying Out Project] Seoul National University Bundang Hospital
[Research Period] March 1, 2021 to February 28, 2026

## Claims

1. A prodrug compound selected from the group consisting of a compound represented by Formula 1 below, a pharmaceutically acceptable salt thereof, a solvate thereof, and a hydrate thereof: 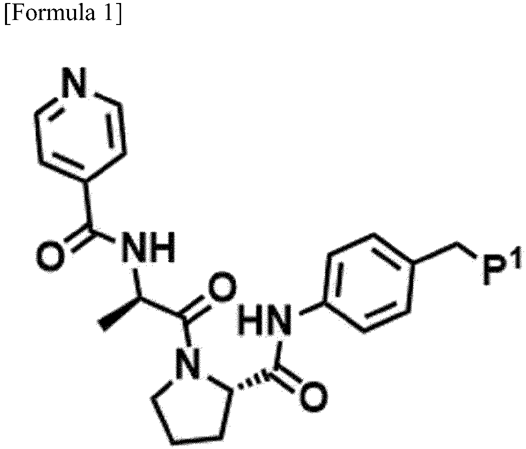 in Formula 1 above, P¹ is a radical of a kinase inhibitor drug containing an oxindole ring, which is a radical derived by removal of a hydrogen atom from an NH group of the oxindole ring.

2. The prodrug compound of claim 1, wherein P¹ is selected from among radicals represented by the following formulas:

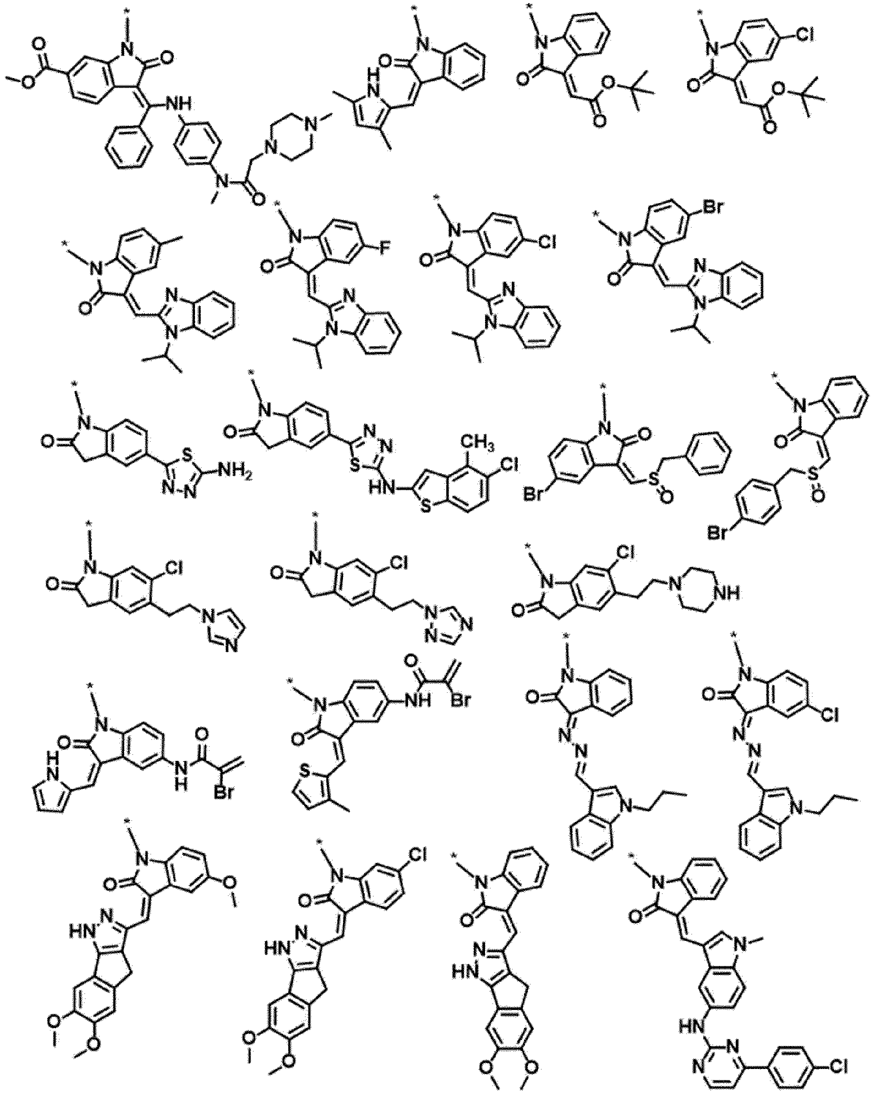

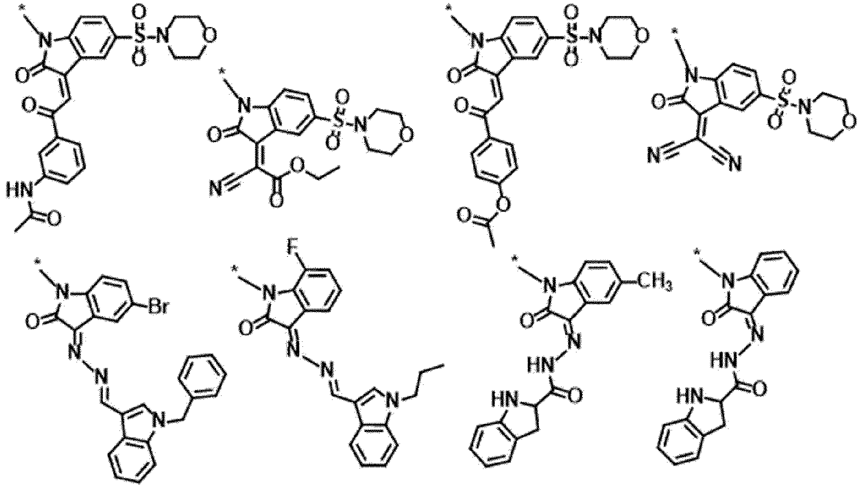

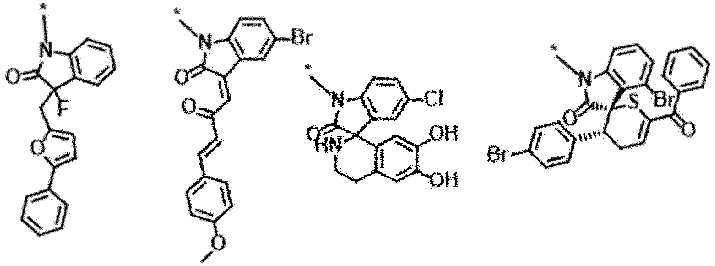

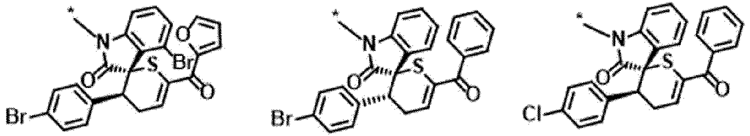

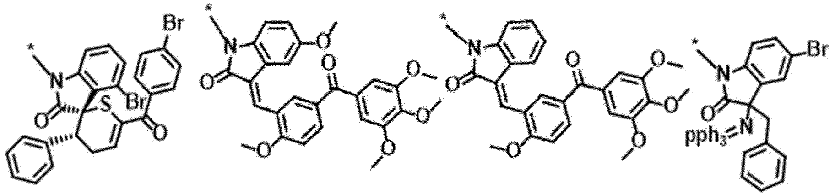

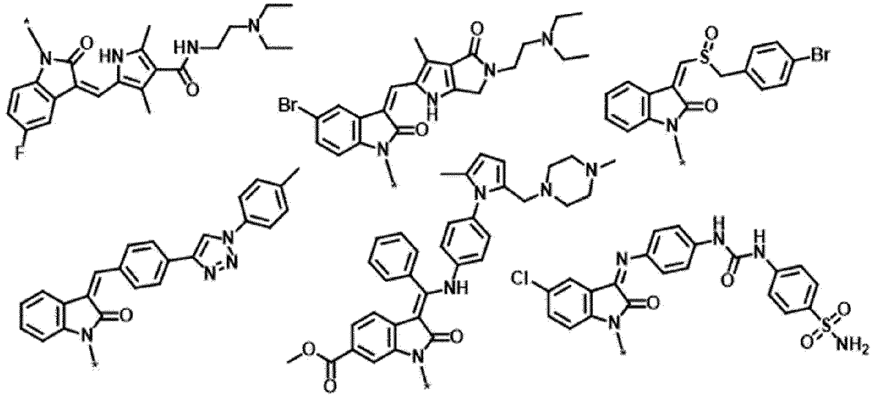

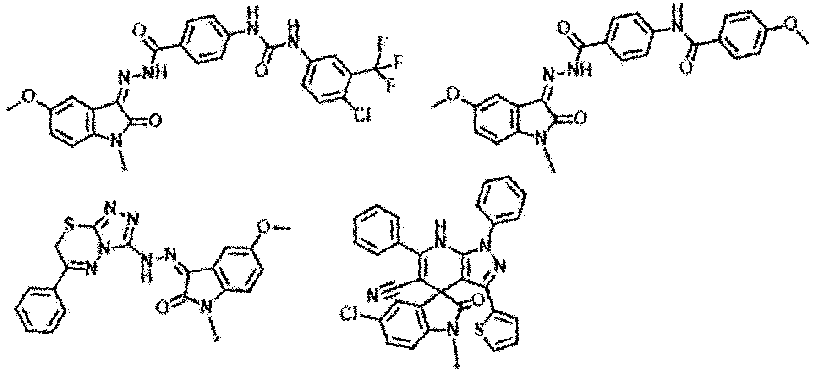

3. The prodrug compound of claim 1, wherein the prodrug compound is represented by Formula 8 below:

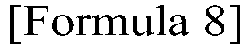

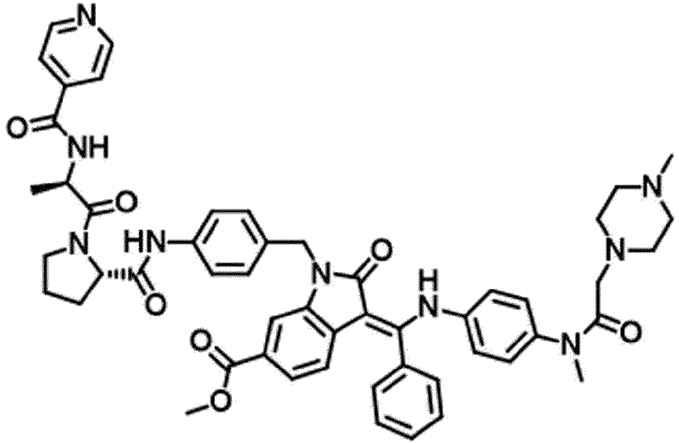

4. The prodrug compound of claim 1, wherein the prodrug compound is degraded by fibroblast activation protein (FAP), so that P¹ is activated.

5. A pharmaceutical composition for preventing or treating fibrotic disease, comprising the prodrug compound of any one of claims 1 to 4 as an active ingredient.

6. The pharmaceutical composition of claim 5, wherein the fibrotic disease is scleroderma, atherosclerosis, cardiac fibrosis, organ transplant fibrosis, muscular fibrosis, pancreatic fibrosis, myelofibrosis, hepatic fibrosis, splenic fibrosis, pulmonary fibrosis, idiopathic pulmonary fibrosis, idiopathic interstitial fibrosis, diffuse interstitial fibrosis, interstitial lung disease, chronic interstitial lung disease, pneumoconiosis, silicosis, interstitial fibrosis, sarcoidosis, mediastinal fibrosis, cardiac fibrosis, atrial fibrosis, endocardial fibrosis, renal fibrosis, macular degeneration, keloid lesions, hypertrophic scars, nephrogenic systemic fibrosis, injection fibrosis, fibrotic complications of surgery, fibrotic chronic allograft vasculopathy, ischemic reperfusion injury-associated fibrosis, arthrofibrosis, Dupuytren's disease, fibrous proliferative lesions of the oral cavity, fibrotic intestinal stenosis, glial scar formation, leptomeningeal fibrosis, fibrosis due to radiation exposure, fibrosis due to mammary cystic rupture, myelofibrosis, retroperitoneal fibrosis, or progressive massive fibrosis.

7. A method for preventing or treating fibrotic disease, comprising a step of administering a pharmaceutically effective amount of the prodrug compound of any one of claims 1 to 4 to a subject in need of administration.

8. The method of claim 7, wherein the fibrotic disease is scleroderma, atherosclerosis, cardiac fibrosis, organ transplant fibrosis, muscular fibrosis, pancreatic fibrosis, myelofibrosis, hepatic fibrosis, splenic fibrosis, pulmonary fibrosis, idiopathic pulmonary fibrosis, idiopathic interstitial fibrosis, diffuse interstitial fibrosis, interstitial lung disease, chronic interstitial lung disease, pneumoconiosis, silicosis, interstitial fibrosis, sarcoidosis, mediastinal fibrosis, cardiac fibrosis, atrial fibrosis, endocardial fibrosis, renal fibrosis, macular degeneration, keloid lesions, hypertrophic scars, nephrogenic systemic fibrosis, injection fibrosis, fibrotic complications of surgery, fibrotic chronic allograft vasculopathy, ischemic reperfusion injury-associated fibrosis, arthrofibrosis, Dupuytren's disease, fibrous proliferative lesions of the oral cavity, fibrotic intestinal stenosis, glial scar formation, leptomeningeal fibrosis, fibrosis due to radiation exposure, fibrosis due to mammary cystic rupture, myelofibrosis, retroperitoneal fibrosis, or progressive massive fibrosis.
